# EUROPEAN PATENT SPECIFICATION

(11) **EP 2 079 510 B1**
(45) Date of publication and mention of the grant of the patent: **31.07.2013**
(21) Application number: 07853715.6
(22) Date of filing: 01.10.2007
(51) Int. Cl.: A61N 1/05

(54) **ELECTRODE ASSEMBLY FOR A STIMULATING MEDICAL DEVICE**
ELEKTRODENANORDNUNG FÜR EINE MEDIZINISCHE STIMULATIONSVORRICHTUNG
ENSEMBLE D'ELECTRODES POUR DISPOSITIF MEDICAL DE STIMULATION

(30) Priority: 29.09.2006 US 529269
(43) Date of publication of application: 22.07.2009
(73) Proprietor: Cochlear Limited, Macquarie University, NSW 2109 (AU)
(72) Inventor: RISI, Frank, Lane Cove, New South Wales 2066 (AU)
(74) Representative: Grünecker, Kinkeldey, Stockmair & Schwanhäusser
(86) International application number: PCT/US2007/080109
(87) International publication number: WO 2008/042863

(56) References cited:
- US-A1- 2004 172 118
- US-A1- 2004 172 118
- US-B1- 6 321 125
- US-B1- 6 498 954
- US-B1- 6 549 814
- US-B1- 6 968 238
- US-B1- 6 968 238
- US-B1- 7 050 858

## Description

### BACKGROUND

### Field of the Invention

The present invention relates generally to stimulating medical devices and, more particularly, to an electrode assembly for a stimulating medical device.

### Related Art

Hearing loss, which may be due to many different causes, is generally of two types, conductive and sensorineural. In some cases, a person may have hearing loss of both types. Conductive hearing loss occurs when the normal mechanical pathways for sound to reach the hair cells in the cochlea are impeded, for example, by damage to the ossicles. Conductive hearing loss is often addressed with conventional hearing aids which amplify sound so that acoustic information can reach the cochlea.

In many people who are profoundly deaf, however, the reason for their deafness is sensorineural hearing loss. This type of hearing loss is due to the absence or destruction of the hair cells in the cochlea which transduce acoustic signals into nerve impulses. Those suffering from sensorineural hearing loss are thus unable to derive suitable benefit from conventional hearing aids due to the damage to or absence of the mechanism for naturally generating nerve impulses from sound.

It is for this purpose that another type of auditory prosthesis, a cochlear™ implant (also commonly referred to as cochlear™ prostheses, cochlear™ devices, cochlear™ implant devices, and the like; generally and collectively referred to herein as "cochlear implants") has been developed. Stimulating auditory prostheses such as cochlear implants bypass the hair cells in the cochlea, directly delivering electrical stimulation to the auditory nerve fibers via an implanted electrode assembly. This enables the brain to perceive a hearing sensation resembling the natural hearing sensation normally delivered to the auditory nerve.

Despite the enormous benefits offered by cochlear implants, one potential disadvantage is that the implanted electrode carrier member is located within the internal canals of the cochlea, generally the scala tympani. Breaching the scala tympani may adversely affect the hydrodynamic behavior of the cochlea and/or damage existing hair cells thereby preventing or at least reducing the likelihood that any residual hearing will be preserved. This may be problematic for those persons who would benefit from use of a cochlear implant to improve hearing of relatively high frequency sound but who have some residual hearing of relatively low frequency sound. In such a case, the recipient is forced to decide whether it will be beneficial to sacrifice any existing residual capacity to hear relatively low frequency sounds to attain the benefits of a cochlear implant to provide hearing sensation of relatively high frequency sounds.

US-B1-6 968 238 relates to a method for inserting a cocklear electrode and an insertion tool and discloses all of the features in the preamble of claim 1.

### SUMMARY

In accordance with one embodiment of the present invention, an electrode assembly for implantation in a cochlea of a recipient according to claim 1 is provided, comprising: a flexible, elongate electrode carrier; and an elongate guide tube having interior dimensions defining a lumen that slidingly retains the carrier, and exterior dimensions that enable the guide tube to be inserted into the recipient's cochlea, the guide tube comprising a rigid tube body and a distal end region that is sufficiently flexible to adjust its shape in response to forces applied by the electrode carrier positioned within its lumen., wherein the distal end region serves as a deployment ramp that causes the electrode carrier to follow a trajectory as it exits the guide tube that is approximately tangential with the surface of the modiolus.

### BRIEF DESCRIPTION OF THE DRAWINGS

Embodiments of the present invention are described below with reference to the attached drawings, in which:

FIG. 1 is a perspective view of an implanted cochlear implant in accordance with one embodiment of the present invention;

FIG. 2A is a perspective, partially cut-away view of a cochlea exposing the canals and nerve fibers of the cochlea;

FIG. 2B is a cross-sectional view of one turn of the canals of a human cochlea;

FIG. 3A is a side view of an electrode assembly in accordance with one embodiment of the present invention shown with the electrode carrier partially extending from the guide tube;

FIG. 3B is a perspective view of an electrode assembly shown in FIG. 3A;

FIG. 3C is a front view of an electrode assembly shown in FIG. 3A;

FIG. 4A is a schematic view of an embodiment of the electrode assembly of the present invention during implantation in a cochlea;

FIG. 4B is a schematic view of the electrode assembly illustrated in FIG. 4A shown at a time during implantation that is later in time relative to the depiction in FIG. 4A;

FIG. 4C is a schematic view of the electrode assembly illustrated in FIG. 4A shown at a time during implantation that is later in time relative to the depiction in FIG. 4B;

FIG. 4D is a schematic view of the electrode assembly illustrated in FIG. 4A shown at a time during implantation that is later in time relative to the depiction in FIG. 4C;

FIG. 4E is a schematic view of the electrode assembly illustrated in FIG. 4A shown at a time during implantation that is later in time relative to the depiction in FIG. 4D;

FIG. 5 is a schematic side view of one embodiment of an electrode assembly of the present invention that facilitates placement of the electrode carrier at a desired depth in the cochlea;

FIG. 6A is a schematic side view of an elongate electrode carrier assembly having a tip in accordance with one embodiment of the present invention shown during implantation;

FIG. 6B is a schematic side view of an elongate electrode carrier assembly of FIG. 6A shown at some later point during implantation;

FIG. 7A depicts longitudinal and lateral cross-sectional views of one embodiment of a cochlear assembly in accordance with one embodiment of the present invention shown in an arrangement prior to implantation;

FIG. 7B depicts longitudinal and lateral cross-sectional views of one embodiment of a cochlear assembly in accordance with one embodiment of the present invention shown in an arrangement that occurs during implantation;

FIG. 7C is a perspective view of a tapered guide tube shown in a flat and rolled configuration, in accordance with one embodiment of the present invention;

FIG. 8A is a perspective view of electrode assembly guide tube in accordance with one embodiment of the present invention;

FIG. 8B is a perspective view of electrode assembly guide tube in accordance with one embodiment of the present invention;

FIG. 8C is a perspective view of electrode assembly guide tube in accordance with one embodiment of the present invention;

FIG. 9 is a perspective view of electrode assembly guide tube in accordance with one embodiment of the present invention;

FIG. 10A is a perspective view of an alternative embodiment of a guide tube of the present invention;

FIG. 10B is a perspective view of an alternative embodiment of a guide tube of the present invention;

FIG. 11 is a perspective view of an alternative embodiment of a guide tube of the present invention;

FIG. 12 is a perspective view of an alternative embodiment of a guide tube of the present invention;

FIG. 13 is a perspective view of the guide tube illustrated in FIG. 12 inserted into a relatively larger cochlea, in accordance with one embodiment of the present invention;

FIG. 14 is a perspective view of the guide tube illustrated in FIG. 12 inserted into a relatively smaller cochlea, in accordance with one embodiment of the present invention;

FIG. 15A is a side view of an embodiment of the guide tube of the present invention not having a carrier-adjustable distal end region implanted in relatively larger cochlea;

FIG. 15B is a side view of an embodiment of the guide tube of the present invention illustrated in FIG. 15A, with the electrode carrier extending from the distal end of the guide tube to bend and contact the modiolus of the cochlea at an angle that likely causes trauma to the cochlea;

FIG. 16A is a side view of an embodiment of the guide tube of the present invention not having a carrier-adjustable distal end region implanted in relatively smaller cochlea;

FIG. 16B is a side view of an embodiment of the guide tube of the present invention illustrated in FIG. 16A, with the electrode carrier extending from the distal end of the guide tube to bend and fold over upon itself;

FIG. 17 is a cross-sectional view of another embodiment of the guide tube of the present invention having a rigid proximal tube body and a carrier-adjustable distal end region;

FIG. 18 is a perspective view of an alternative embodiment of the guide tube illustrated in FIG. 17;

FIG. 19 is a perspective view of an alternative embodiment of the guide tube illustrated in FIG. 17;

FIG. 20 is a perspective view of an alternative embodiment of the guide tube illustrated in FIG. 17;

FIG. 21 is a perspective view of an alternative embodiment of the guide tube illustrated in FIG. 17;

FIG. 22 is a perspective view of an alternative embodiment of the guide tube illustrated in FIG. 17;

FIG. 23 is a perspective view of an alternative embodiment of the guide tube illustrated in FIG. 17;

FIG. 24 is a perspective view of an alternative embodiment of the guide tube illustrated in FIG. 17;

FIG. 25A is a perspective view of the guide tube illustrated in FIG. 17 inserted into an average size cochlea, in accordance with one embodiment of the present invention;

FIG. 25B is a perspective view of the guide tube illustrated in FIG. 17 inserted into an average size cochlea and in which the electrode carrier is extended through the guide tube such that the distal end of the electrode carrier is positioned exiting the distal end of the carrier-adjustable distal end region of the guide tube thereby causing the distal end region to bend slightly to come into contact with the modiolus of the cochlea, in accordance with one embodiment of the present invention;

FIG. 25C is a perspective view of the guide tube illustrated in FIG. 17 inserted into an average size cochlea and in which the electrode carrier is extended through the guide tube such that the distal end of the electrode carrier is positioned substantially beyond the distal end of the carrier-adjustable distal end region of the guide tube to be atraumatically positioned adjacent the modiolus of the cochlea, in accordance with one embodiment of the present invention;

FIG. 26A is a perspective view of the guide tube illustrated in FIG. 17 inserted into a relatively smaller cochlea, in accordance with one embodiment of the present invention;

FIG. 26B is a perspective view of the guide tube illustrated in FIG. 17 inserted into the relatively smaller cochlea illustrated in FIG. 26A, and in which the electrode carrier is extended through the guide tube such that the distal end of the electrode carrier is positioned exiting the distal end of the carrier-adjustable distal end region of the guide tube thereby causing the distal end region to bend somewhat more than in FIG. 25B to come into contact with the modiolus of the cochlea, in accordance with one embodiment of the present invention;

FIG. 26C is a perspective view of the guide tube illustrated in FIG. 17 inserted into the relatively smaller cochlea illustrated in FIG. 26A and in which the electrode carrier is extended through the guide tube such that the distal end of the electrode carrier is positioned substantially beyond the distal end of the carrier-adjustable distal end region of the guide tube to be atraumatically positioned adjacent the modiolus of the cochlea, in accordance with one embodiment of the present invention;

FIG. 27A is a perspective view of the guide tube illustrated in FIG. 17 inserted into a relatively larger cochlea, in accordance with one embodiment of the present invention;

FIG. 27B is a perspective view of the guide tube illustrated in FIG. 17 inserted into the relatively larger cochlea illustrated in FIG. 27A, and in which the electrode carrier is extended through the guide tube such that the distal end of the electrode carrier is positioned exiting the distal end of the carrier-adjustable distal end region of the guide tube thereby causing the distal end region to bend somewhat to come into contact with the modiolus of the cochlea, in accordance with one embodiment of the present invention;

FIG. 27C is a perspective view of the guide tube illustrated in FIG. 17 inserted into the relatively smaller cochlea illustrated in FIG. 27A and in which the electrode carrier is extended through the guide tube such that the distal end of the electrode carrier is positioned substantially beyond the distal end of the carrier-adjustable distal end region of the guide tube to be atraumatically positioned adjacent the modiolus of the cochlea, in accordance with one embodiment of the present invention;

FIG. 28A is a side view of a relatively larger cochlea with an alternative embodiment of a guide tube of the present invention having position markers; and

FIG. 28B is a side view of a smaller larger cochlea with an alternative embodiment of a guide tube of the present invention having position markers.

### DETAILED DESCRIPTION

Embodiments of the present invention are generally directed to an electrode assembly comprising a low-profile, low-volume elongate electrode carrier and a corresponding guide tube for introducing the carrier into the cochlea to place electrodes disposed at the distal end of the carrier at desired locations along the tonotopically-mapped cochlea. Embodiments of the electrode assembly of the present invention facilitate intra- and extra-cochlea atraumatic implantation of the unobtrusive electrode carrier of the present invention thereby minimizing adverse impact to natural auditory functioning. For example, as will be described in detail below, an electrode assembly of the present invention may be utilized to implant a carrier of the present invention into the scala tympani with minimal damage to the delicate structures of the cochlea and without substantial interference with the natural hydrodynamic nature of the cochlea such as the natural flow of perilymph in the cochlea canals. In one particular embodiment, the carrier is pre-curved to attain a perimodiolar position in the scala tympani to facilitate accurate delivery of electrical stimulation with a minimum stimulation current and power consumption.

Embodiments of the electrode assembly of the present invention may be used to provide therapeutic benefits in a variety of applications. For example, the present invention may be utilized to improve the hearing of relatively high frequencies in those recipients who have residual hearing of relatively low frequencies. The spiral ganglion and other cells responsible for the perception of high frequency sounds are generally located at the basal end of the cochlea. For those individuals who suffer from high frequency hearing loss, the hair cells in the basal region of the cochlea are ineffective or otherwise damaged. In such applications, cochlear implants utilizing a carrier of the present invention provide direct electrical stimulation of the basal nerve cells, thereby enhancing the hearing of high frequency sounds, while simultaneously relying on the recipient's residual hearing to sense low-to-mid-frequency sounds. This makes the electrode assembly of the present invention particularly beneficial when used in connection with stimulating auditory prostheses that are utilized as part of an electro-acoustic stimulation (EAS) device.

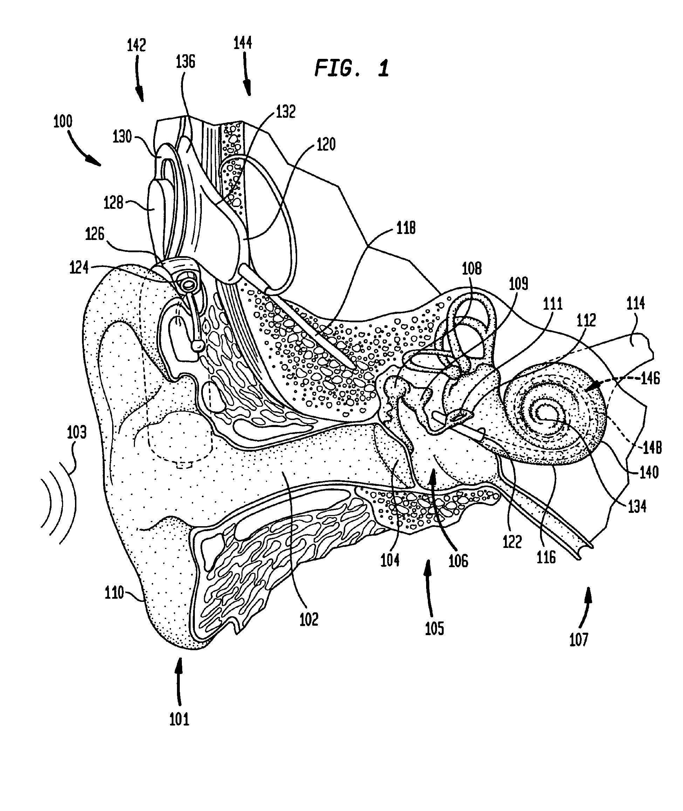

FIG. 1 is perspective view of one embodiment of a cochlear implant 100 implanted in a human recipient. Referring now to FIG. 1, the relevant components of outer ear 101, middle ear 105 and inner ear 107 are described next below. A fully functional ear outer ear 101 comprises an auricle 110 and an ear canal 102. An acoustic pressure or sound wave 103 is collected by auricle 110 and channeled into and through ear canal 102. Disposed across the distal end of ear cannel 102 is a tympanic membrane 104 which vibrates in response to sound wave 103. This vibration is coupled to oval window or fenestra ovalis 112 through three bones of middle ear 105, collectively referred to as the ossicles 106 and comprising the malleus 108, the incus 109 and the stapes 111. Bones 108, 109 and 111 of middle ear 105 serve to filter and amplify sound wave 103, causing oval window 112 to articulate, or vibrate. Such vibration sets up waves of fluid motion within cochlea 140. Such fluid motion, in turn, activates tiny hair cells (not shown) that line the inside of cochlea 140. Activation of the hair cells causes appropriate nerve impulses to be transferred through the spiral ganglion cells and auditory nerve 114 to the brain, where they are perceived as sound.

Cochlear implant 100 comprises external component assembly 142 which is directly or indirectly attached to the body of the recipient, and an internal component assembly 144 which is temporarily or permanently implanted in the recipient. External assembly 142 typically comprises microphone 124 for detecting sound, a speech processing unit 126, a power source (not shown), and an external transmitter unit 128. External transmitter unit 128 comprises an external coil 130 and, preferably, a magnet (not shown) secured directly or indirectly to external coil 130. Speech processing unit 126 processes the output of microphone 124 that is positioned, in the depicted embodiment, by auricle 110 of the recipient. Speech processing unit 126 generates coded signals, referred to herein as a stimulation data signals, which are provided to external transmitter unit 128 via a cable (not shown).

Internal assembly 144 comprises an internal receiver unit 132, a stimulator unit 120, and an elongate electrode carrier 118. Internal receiver unit 132 comprises an internal transcutaneous transfer coil 136, and preferably, a magnet (also not shown) fixed relative to the internal coil. Internal receiver unit 132 and stimulator unit 120 are hermetically sealed within a biocompatible housing. The internal coil receives power and stimulation data from external coil 130, as noted above. Elongate electrode carrier 118 has a proximal end connected to stimulator unit 120 and extends from stimulator unit 120 to cochlea 140. Electrode carrier 118 is implanted into cochlea 104 via a cochleostomy 122.

Electrode carrier 118 comprises an electrode array 146 disposed at the distal end thereof. Electrode array 146 comprises a plurality of longitudinally-aligned electrodes 148. Stimulation signals generated by stimulator unit 120 are applied by electrodes 148 to cochlea 140, thereby stimulating auditory nerve 114.

In one embodiment, external coil 130 transmits electrical signals (i.e., power and stimulation data) to the internal coil via a radio frequency (RF) link. The internal coil is typically a wire antenna coil comprised of multiple turns of electrically insulated single-strand or multi-strand platinum or gold wire. The electrical insulation of the internal coil is provided by a flexible silicone molding (not shown). In use, implantable receiver unit 132 may be positioned in a recess of the temporal bone adjacent auricle 101 of the recipient.

There are several speech coding strategies that may be implemented by speech processor 126 to convert sound 103 into an electrical stimulation signal. Embodiments of the present invention may be used in combination with any speech strategy now or later developed, including but not limited to Continuous Interleaved Sampling (CIS), Spectral PEAK Extraction (SPEAK), Advanced Combination Encoders (ACE), Simultaneous Analog Stimulation (SAS), MPS, Paired Pulsatile Sampler (PPS), Quadruple Pulsatile Sampler (QPS), Hybrid Analog Pulsatile (HAPs), n-of-m and HiRes™, developed by Advanced Bionics. SPEAK is a low rate strategy that may operate within the 250-500 Hz range. ACE is a combination of CIS and SPEAK. Examples of such speech strategies are described in U.S. Patent No. 5,271,397. The present invention may also be used with other speech coding strategies, such as a low rate strategy called Spread of Excitation which is described in U.S. Provisional No. 60/557,675 entitled, "Spread Excitation and MP3 coding Number from Compass UE" filed on March 31, 2004, U.S. Provisional No. 60/616,216 entitled, "Spread of Excitation And Compressed Audible Speech Coding" filed on October 7, 2004 (corresponding US Patents: US 7822478 and US 8285382) and PCT Application WO 02/17679A1, entitled "Power Efficient Electrical Stimulation."

Embodiments of cochlear implant 100 may locally store several speech strategies, such as in the form of a software program or otherwise, any one of which may be selected depending, for example, on the aural environment. For example, a recipient may choose one strategy for a low noise environment, like a conversation in an enclosed room, and second strategy for a high noise environment, like on a public street. The programmed speech strategies may be different versions of the same speech strategy, each programmed with different parameters or settings.

The successful operation of cochlear implant 100 depends in part on its ability to convey pitch information. Differing pitch percepts may be produced by cochlear implant 100 in two distinct ways. First, electrical stimulation at different sites in cochlea 140 excites different groups of neurons and because of the tonotopic arrangement of neurons in cochlea 140, different pitch sensations result. The term "tonotopic" is meant that the percept corresponding to a particular site in the cochlea changes in pitch from lower to higher as the site is changed in an apical 134 to basal 116 direction. Pitch varied in this way is known as "place pitch." Secondly different pulse rates of electrical stimulation produce different pitch sensations. Pitch varied in this way is known as "rate pitch."

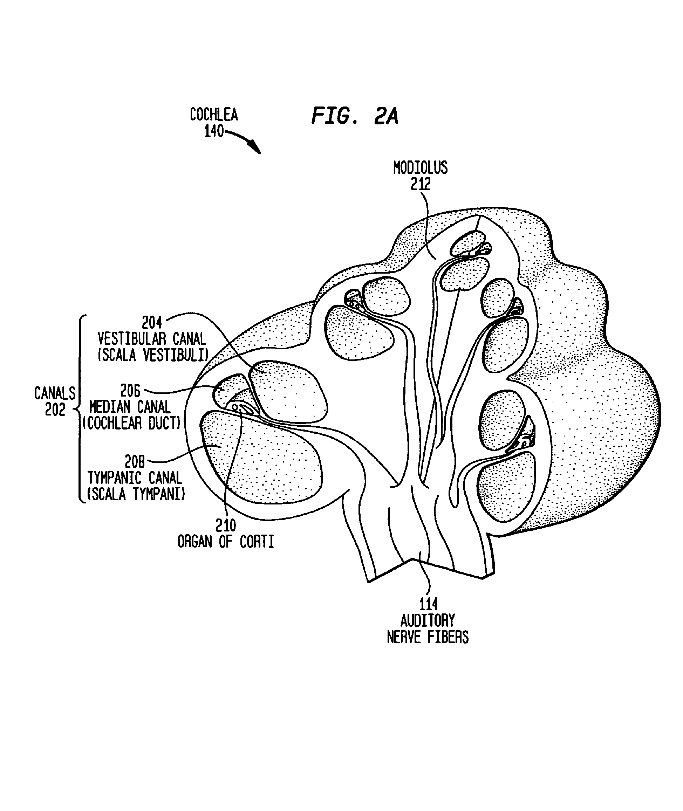

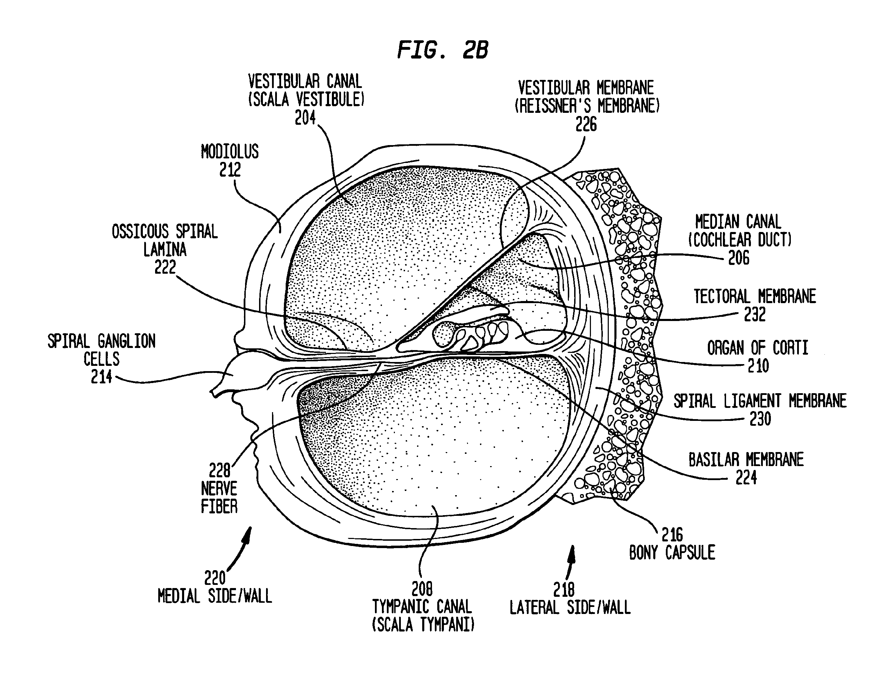

Relevant aspects of a human cochlea are described next below with reference to FIGS. 2A and 2B. FIG. 2A is a perspective view of a human cochlea partially cut-away to display the canals and nerve fibers of the cochlea. FIG. 2B is a cross-sectional view of one turn of the canals of the cochlea illustrated in FIG. 2A. To facilitate understanding, the following description will reference the cochlea illustrated in FIGS. 2A and 2B as cochlea 140, which was introduced above with reference to FIG. 1, and which is referenced below. It should be appreciated that embodiments of the present invention may be implanted in any cochlea to provide therapeutic benefits for a variety ailments now or later discovered.

Referring to FIG. 2A, cochlea 140 is a conical spiral structure comprising three parallel fluid-filled canals, one or more of which are sometimes referred to as ducts. The canals, collectively and generally referred to herein as canals 202, comprise the tympanic canal 208, also know as the scala tympani 208, the vestibular canal 204, also referred to as the scala vestibule 204, and the median canal 206, also referred to as the cochlear duct 206. Cochlea 140 has a conical shaped central axis, the modiolus 212, that forms the inner wall of scala vestibule 204 and scala typani 208. Tympanic and vestibular canals 208, 204 transmit pressure, while medial canal 206 contains the organ of Corti 210 which detects pressure impulses and responds with electrical impulses which travel along spiral ganglion cells 214 to the auditory nerve fibers 114 to the brain (not shown).

Referring now to FIG. 2B, separating canals 202 of cochlea 140 are various membranes and other tissue. The Ossicous spiral lamina 222 projects from modiolus 212 to separate scala vestibule 204 from scala tympani 208. Toward lateral side 218 of scala tympani 208, a basilar membrane 224 separates scala tympani 208 from cochlear duct 206. Similarly, toward lateral side 218 of scala vestibule 204, a vestibular membrane 226, also referred to as the Reissner's membrane 226, separates scala vestibule 204 from cochlear duct 206.

The fluid in tympanic and vestibular canals 208, 204, referred to as perilymph, has different properties than that of the fluid which fills cochlear duct 206 and surrounds organ of Corti 210, referred to as endolymph. Sound entering auricle 110 causes pressure changes in cochlea 140 to travel through the fluid-filled tympanic and vestibular canals 208, 204. As noted, organ of Corti 210 is situated on basilar membrane 224 in cochlear duct 206. It contains rows of 16,000-20,000 hair cells (not shown) which protrude from its surface. Above them is the tectoral membrane 232 which moves in response to pressure variations in the fluid-filled tympanic and vestibular canals 208, 204. Small relative movements of the layers of membrane 232 are sufficient to cause the hair cells to send a voltage pulse or action potential down the associated nerve fiber 228. Nerve fibers 228, embedded within spiral lamina 222, connect the hair cells with the spiral ganglion cells 214 which form auditory nerve fibers 114. These impulses travel to the auditory areas of the brain for processing.

The place along basilar membrane 224 where maximum excitation of the hair cells occurs determines the perception of pitch and loudness according to the place theory. Due to this anatomical arrangement, cochlea 140 has characteristically been referred to as being "tonotopically mapped." This property of cochlea 140 has traditionally been exploited by longitudinally positioning electrodes 148 along carrier 118 to deliver to a selected region within scala tympani 208 a stimulating signal within a predetermined frequency range.

Portions of cochlea 140 are encased in a bony capsule 216. Referring to FIG. 2B, cochlear bony capsule 216 resides on lateral side 218 (the right side as drawn in FIG. 2B), of cochlea 140. Spiral ganglion cells 214 reside on the opposing medial side 220 (the left side as drawn in FIG. 2B) of cochlea 140. A spiral ligament membrane 230 is located between lateral side 218 of scala tympani 208 and bony capsule 216, and between lateral side 218 of cochlear duct 206 and bony capsule 216. Spiral ligament 230 also typically extends around at least a portion of lateral side 218 of scala vestibuli 204.

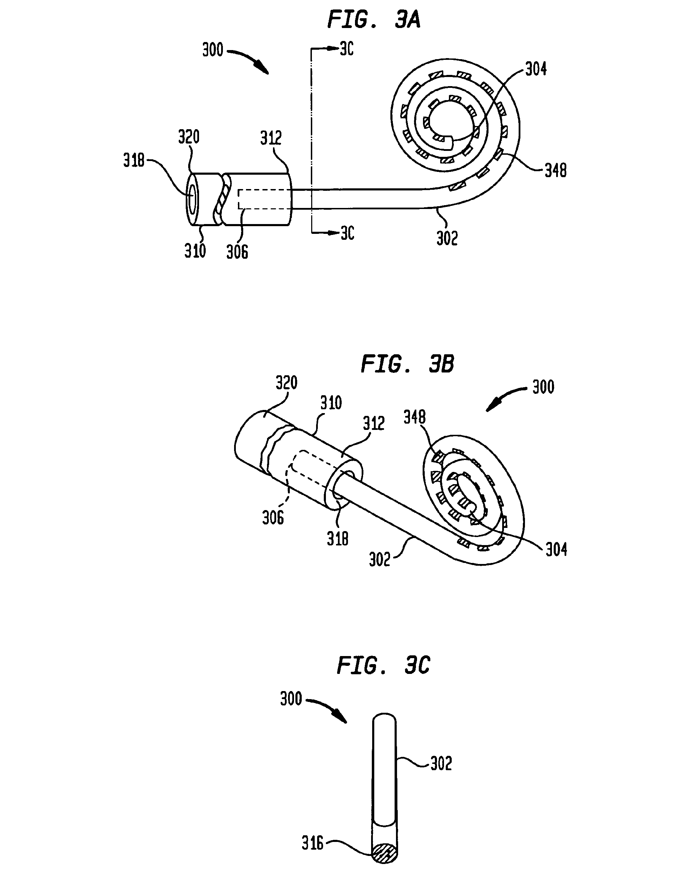

FIGS. 3A and 3B are side and perspective views, respectively, of one embodiment of an electrode assembly of the present invention, referred to herein as electrode assembly 300. Electrode assembly 300 comprises an embodiment of elongate low-profile, low-volume electrode carrier 118, referred to herein as electrode carrier 302 having a distal end 304 and a proximal end 306. A guide tube 310 is shown in FIGS. 3A and 3B surrounding a proximal region of electrode carrier 302. In FIGS. 3A and 3B carrier 302 is illustrated extending from distal end 312 of guide tube 310 at which point carrier 302 takes on a coiled configuration because this embodiment of carrier 302 is precurved in a manner disclosed elsewhere herein. It should also be appreciated that electrode carrier 302 has a plurality of electrodes 148, referred to herein as electrodes 348, disposed toward apical end 304 of carrier 302. In the embodiment shown in FIGS. 3A-3C, electrodes 348 are longitudinally-spaced to align with specific regions of tonotopically-mapped cochlea 140. FIG. 3C is a cross-sectional view of the embodiment of electrode carrier 302 taken along section line 3C-3C in FIG. 3A.

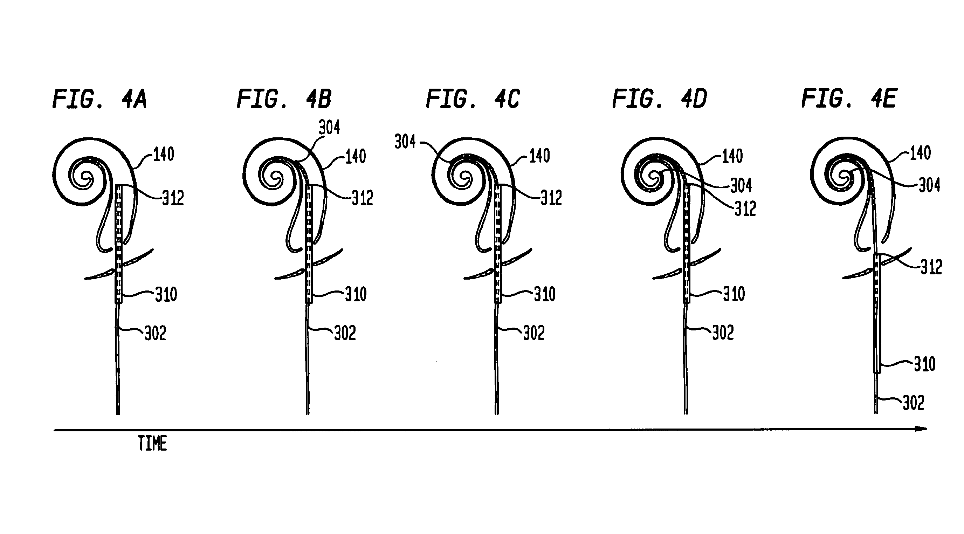

FIGS. 4A-4E are a series of side-views showing consecutive events occurring during implantation of electrode carrier 302 of electrode assembly 300. Initially, electrode carrier 302 and guide tube 310 are assembled; that is, electrode carrier 302 is slidingly inserted into lumen 318 of guide tube 310. The combined arrangement is then inserted to a predetermined depth into cochlea 140, as illustrated in FIG. 4A. Typically, such an introduction to cochlea 140 is achieved via cochleostomy 122 (FIG. 1) or through fenestra ovalis 112 (FIG. 1). In the exemplary implantation shown in FIG. 4A, the combined arrangement of carrier 302 and tube 310 is inserted to approximately the first turn of cochlea 140.

As shown in FIG. 4A, the combined arrangement of guide tube 310 and electrode carrier 302 is substantially straight. This is due in part to the rigidity of guide tube 310 relative to the force applied to the interior wall of lumen 318 (FIGS. 3A-3B) by precurved carrier 302. This prevents tube 310 from bending or curving in response to forces applied by carrier 302.

This is advantageous over conventional electrode carriers that require the use of a stylet or other positioner ("stylet" herein) to introduce the carrier in to cochlea 140. As is well known to those of ordinary skill in the art, the combined conventional arrangement of a stylet and a conventional carrier member is typically curved prior to implantation. This is because such stylets are somewhat flexible. Such curvature often results in the carrier pressing up against spiral ligament membrane 230, basilar membrane 224, and other structures of cochlea 140, during implantation. This, of course, increases the likelihood that trauma occurs during implantation.

In addition, due to this flexibility of conventional electrode assemblies, there is a tendancy for conventional electrode carriers to buckle during insertion, particularly when introduced via round window 112 (FIG. 1). Guide tube 310 is preferably sufficiently rigid so that it is capable of withstanding any forces normally experienced during implantation that would cause the device to bend in response to the buckling of electrode carrier 302. Furthermore, lumen 318 of guide tube 302 has an inner diameter that is just slightly greater than the outer diameter of elongate carrier 302 thereby minimizing the space between carrier 302 and guide tube 310. This prevents elongate carrier 302 from buckling during implantation. Rather, buckling forces are transferred to rigid guide tube 310. This allows carrier 302 to remain flexible for successful implantation to a desired depth in cochlea 140. It should be appreciated that the rigidity of guide tube 310 may be determined based on other factors as well, such as the ability to bend or curve subsequent to initial implantation but desirable to have distal end 312 of guide tube 310 to reach a desired depth in, for example, cochlea 140.

In this exemplary embodiment, electrode assembly 300 is configured to place stimulating electrodes 148 as close as possible to modiolus 212 and, therefore, ganglion cells 214. To attain this, this embodiment of electrode carrier 302 is manufactured in a curved configuration; that is, precurved, as noted above. In the embodiment configured to be implanted in scala tympani 208, electrode carrier 302 is precurved to have a radius of curvature that approximates the curvature of medial side 220 of scala tympani 208. Such embodiments of the electrode assembly of the present invention are referred to as perimodiolar electrode assemblies, and this position within cochlea 140 as the perimodiolar position. Advantageously, placing electrodes in the perimodiolar position provides a greater specificity of electrical stimulation, reduces the requisite current levels, and results in lower power consumption.

As shown in FIGS. 4B-4D, after the combined arrangement of carrier 302 and guide tube 310 is inserted to approximately the first turn of cochlea 140, carrier 302 is then continually advanced through guide tube 310 while the guide tube is maintained in a substantially stationary position. This causes distal end 304 of electrode carrier 302 to extend from distal end 312 of guide tube 310. As it does so, the illustrative embodiment of carrier 302 bends or curves to attain a perimoidular position, as shown in FIGS. 4B-4D. Once carrier 302 is located at the desired depth in scala tympani 208, guide tube 310 is removed from cochlea 140 while electrode carrier 302 is maintained in a stationary position. This is illustrated in FIG. 4E.

The control of electrode carrier 302 is provided by guide tube 310, not a stylet as in conventional electrode assemblies. As such, electrode carriers of the present invention such as electrode carrier 302 do not have a lumen to receive a stylet. This is illustrated in the cross-sectional view of FIG. 3C. Further, electrode carriers of the present invention need not be manufactured with the thickness or structural integrity to ensure the electrode carrier is not punctured or torn by the stylet during implantation.

Thus, in contrast to conventional electrode carriers, electrode carriers of the present invention such as carrier 118 (FIG. 1) and 302 (FIGS. 3A-C, 4A-E) are low-profile, low-volume elongate carriers. In the embodiment illustrated in FIG. 3C, carrier 302 is substantially solid with embedded leads 316 for electrodes 148. As such, neither stylets nor positioners are factors considered when determining the physical size, shape or profile of an electrode carrier of the present invention.

In one embodiment, carrier 302 has a thickness or diameter of between approximately 0.35 and 0.55 millimeters along its length, and a diameter of approximately 0.27 millimeters at its distal end 304. In another embodiment, electrode carrier 302 has a medial length of 13.33 millimeters, which is the same as the medial length of a conventional Contour™ electrode carrier member available from Cochlear Limited, Australia. A carrier 302 having the dimensions noted above has a volume of 1.769 square millimeters. This is substantially less than the volume of the noted Contour™ electrode carrier member, which is 8.266 square millimeters. That is, the volume of such an embodiment of the electrode carrier is 21.4% of the volume of a conventional Contour electrode carrier (1.769/8.266 = 0.214). Because the volume of such embodiments of the carrier is substantially less than conventional carriers, such electrode carriers of the present invention are referred to as low-profile electrode carriers. It should be appreciated that this reduction in volume is attained without reducing the quantity of electrodes disposed on the carrier.

In another embodiment, carrier 302 has a thickness or diameter of between approximately 0.25 and 0.65 millimeters along its length, and a diameter of between approximately 0.25 and 0.35 millimeters at its distal end 304. As one of ordinary skill in the art would appreciate, such dimensions are exemplary only, and electrode carriers of the present invention may be provided with other dimensions due to the elimination of a stylet lumen.

The above reduction in carrier volume may be achieved, for example, by reducing the cross-sectional area of the carrier by approximately 50%, either uniformly or non-uniformily, using existing manufacturing technology. As one of ordinary skill in the art would appreciate, the thickness or diameter of electrode carrier 302 is dependent on the selected materials and manufacturing processes, and that carriers much thinner than those noted above may be manufactured, and are considered to be within the scope of the present invention.

It should be appreciated that, as noted, lumen 318 of embodiments of guide tube 310 has a diameter that is suitable to slidingly receive the embodiment of low-profile, low-volume elongate carrier 302 that is implemented in electrode assembly 300. Guide tube 310, as noted, is further configured to introduce carrier 302 into cochlea 140 so as to place electrodes 348 disposed at distal end 304 of carrier 302 at desired locations along tonotopically-mapped cochlea 140. Preferably, guide tube 310 is sufficiently thin to achieve this while facilitating intra- and extra-cochlea atraumatic implantation of carrier 302.

In the embodiments illustrated in FIGS. 3A and 3B, the cross-sectional profile of guide tube 310 is round. It should be appreciated, however, that embodiments of the guide tube of the present invention may have any cross-sectional profile suitable for a particular application. For example, in alternative embodiments, the guide tube has an oval or rectangular cross-sectional profile.

Also, embodiments of the guide tube of the present invention may be constructed from ay suitable material or combination of materials now or later developed that are appropriate or acceptable for a given application. Such materials may include, but are not limited, to, polymers, metals, combination of fixed and bioresorbable polymers, etc. In one embodiment, the guide tube is constructed from one or more bioresorbable materials so that once the electrode carrier is implanted and the guide tube is retracted, the guide tube may remain surrounding the extra-cochlear carrier 302 to be resorbed over a specified period of time. In one embodiment, the bioresorbable guide tube remains within the mastoid cavity to be entirely resorbed extra-cochlearly. As one of ordinary skill in the art would appreciate, the bioresorbable material would be selected to have a specified rate of resorbption such that the guide tube would be completely resorbed after, for example, 2-4 weeks; that is, prior to when cochlear implant 100 is initially powered on. As is also apparent to those of ordinary skill in the art, there are many bioresorbable materials available with a few having proven biocompatability and FDA approval. Examples include but are not limited to those based on PLA (polylactic acid) or PGA (polyglycolic acid).

In one embodiment, the guide tube is constructed from a bioresorbable material that is impregnated or coated with a compound or combination of compounds suitable for achieving one or more desired functions. For example, in some embodiments, an anti-bacterial or antiinflammatory (or other) compound is impregnated in or coated on the guide tube to, for example, prevent infection, reduce fluid accumulation, tissue inflammation, etc.

In alternative embodiments, the guide tube is coated or impregnated to provide one or more other properties in addition to or in the alternative to those identified above. For example, in one embodiment, the guide tube is coated or impregnated with a compound that contributes to the lubricity of the associated electrode carrier. In another embodiment, the guide tube is coated or impregnated with a compound that contributes to drug elution of the implanted elongate low-profile, low-volume electrode carrier.

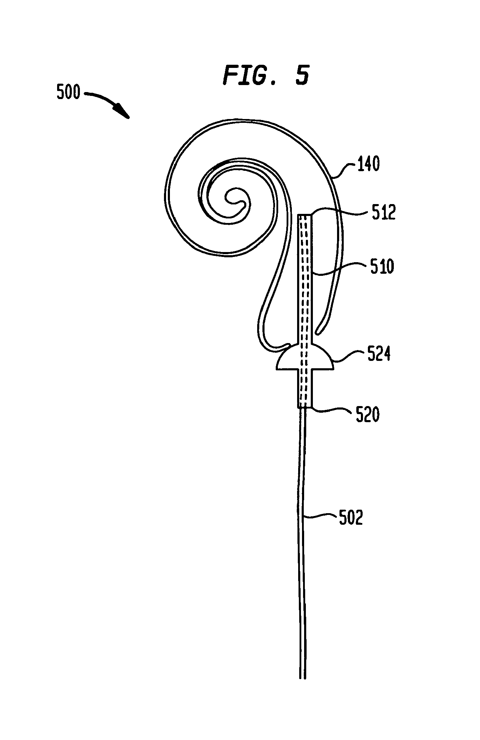

FIG. 5 is a schematic side view of one embodiment of an electrode assembly of the present invention that facilitates placement of an electrode carrier at a desired depth in cochlea 140. The embodiment of the electrode assembly of the present invention illustrated in FIG. 5 is referred to herein as electrode assembly 500. Electrode assembly 500 comprises an embodiment of guide tube 310 (FIG. 3A and 3B), referred to herein as guide tube 510, and an embodiment of elongate low-profile, low-volume electrode carrier 118, referred to herein as carrier 502. In the arrangement illustrated in FIG. 5, electrode carrier 502 is located within guide tube 510, and the combined arrangement has be implanted in cochlea 140 up to the first turn of the cochlea.

Proximal end 520 of guide tube 510 comprises a radially-extending extension 524 that serves as means to assist the audiologist in determining the appropriate depth at which to implant guide tube 502. For example, in the embodiment shown in FIG. 5, guide tube 502 is configured to abut cochlea 140 when distal end 512 of guide tube 510 is located at the first turn of cochlea 140.

As one of ordinary skill in the art would appreciate, extension 524 may have different configurations in alternative embodiments of the present invention. For example, in one alterative embodiment, extension 524 is formed as an extension arm that extends from a relatively small radius on guide tube 510.

In one embodiment, extension 524 is further configured to provide a surface that may be used by the audiologist to grip and position guide tube 510 and carrier 502 during implantation.

In alternative embodiments extension 524 may provide an indication of an insertion depth rather than cease advancement of electrode assembly 500. For example, extension 524 may be implemented to provide visual feedback to the implanting audiologist. For example, in one embodiment, a radial extension 524 is implemented as a marker located on the surface of the guide tube that is visible to the implanting audiologist until the guide tube is inserted into cochlea 140 beyond a desired depth. At that point the marker is no longer visible, indicating to the audiologist that the guide tube is at the desired depth. Alternative embodiments may include multiple markers each indicating a different insertion depth has been achieved. It should be appreciated that an extension, marker and/or other similar elements may be incorporated in any combination in alternative embodiments of the present invention.

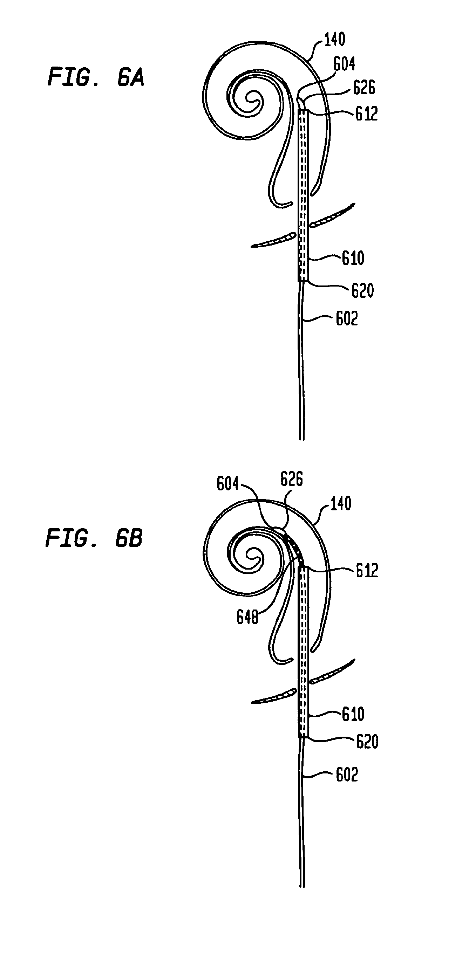

FIG. 6A is a schematic side view of an elongate electrode carrier assembly in accordance with one embodiment of the present invention shown during implantation. FIG. 6B is a schematic side view of an elongate electrode carrier assembly of FIG. 6A shown at some later point during implantation. The electrode assembly shown in FIGS. 6A and 6B, referred to herein as electrode assembly 600, comprises a low-profile, low-volume elongate electrode carrier 602 and an associated guide tube 610, constructed and arranged in accordance with the teachings of the present invention. In this embodiment, distal end 612 of guide tube 610 interacts with distal end 604 of electrode carrier 602 to prevent the sheath from sliding off the carrier; that is, to maintain a relative longitudinal position. In this embodiment, a barb or hooked tip 626 disposed at distal end 602 of carrier 602 prevents the carrier from being accidentally withdrawn from the guide tube during surgery or handling. Barb 626 may also be used to orientate electrode carrier 602. In one embodiment, barb 626 is in the form of a soft silicone extension disposed at distal end 604.

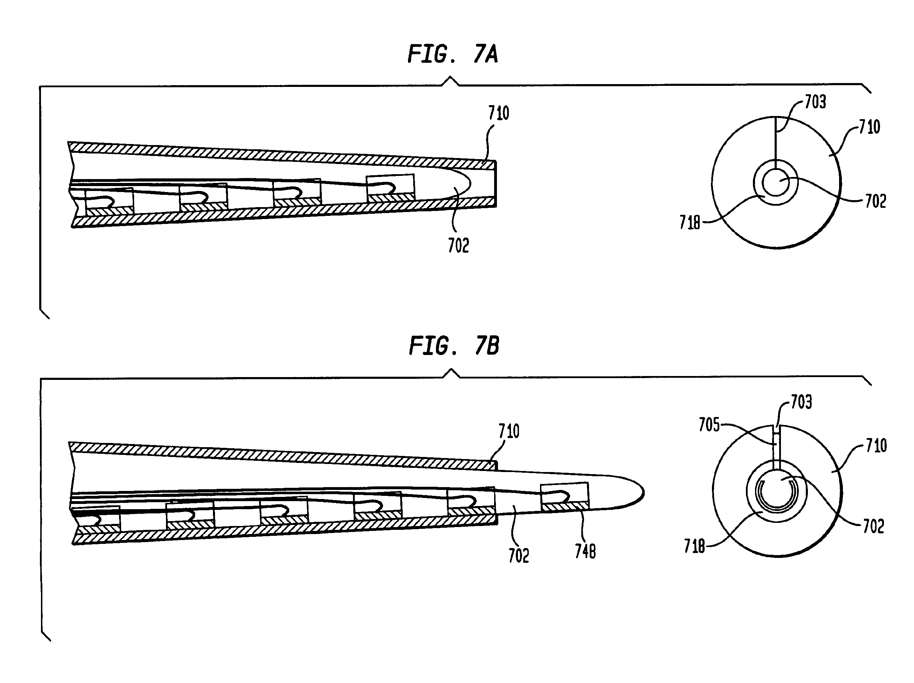

FIG. 7A depicts longitudinal and lateral cross-sectional views of one embodiment of a cochlear assembly in accordance with one embodiment of the present invention shown in an arrangement prior to implantation. FIG. 7B depicts longitudinal and lateral cross-sectional views of the embodiment of the cochlear assembly illustrated in FIG. 7A shown in an arrangement that occurs during implantation. In this embodiment, electrode assembly 700 comprises an embodiment of guide tube 310, referred to herein as guide tube 710, and an embodiment of carriers 118 and 302, referred to herein as electrode carrier 702.

Electrode carrier 702 is, in this embodiment, tapered. Guide tube 710 is made of a flexible material so that it may be tapered to hug the profile of tapered electrode carrier 702, as shown in FIGS. 7A and 7B. The taper could be achieved by having a flexible guide tube that expanded as electrode carrier 702 is slidingly advanced through tube 710.

In the embodiment illustrated in FIGS. 7A and 7B, guide tube 710 has an axial slit 703 to guide electrode carrier 702 which, in this embodiment, has a corresponding mating feature 705. In alternative embodiments, axial slit 703 is further configured to be used to split guide tube 710 for removal, or to allow guide tube 710 to be adapted to an external tool for automated insertion.

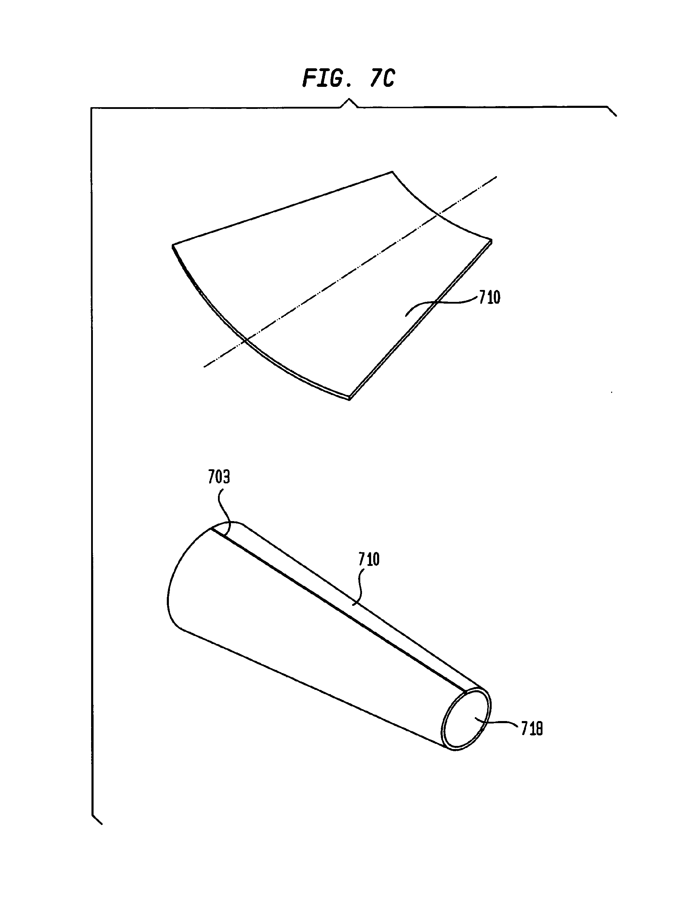

Referring to FIG. 7C, to achieve a tapered guide tube 710 that is also removable, a rolled design may be applied in which a thin flat sheet of material is rolled to a specified tapered profile that will keep electrode carrier 702 straight for insertion, but can also then expand as the carrier is advanced. Once guide tube 710 is withdrawn, it may then be unrolled from the lead connecting carrier 702 to stimulator unit 120 (FIG. 1) and disposed.

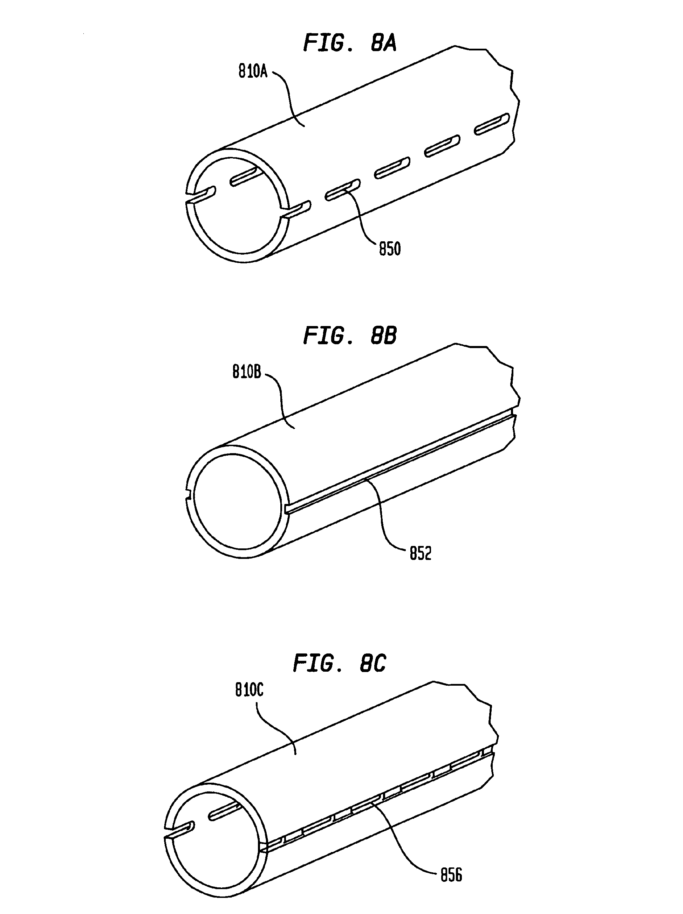

FIGS. 8A-8C are perspective views of alternative embodiments of the guide tube of the present invention, referred to herein as guide tubes 810A, 810B and 810C, respectively. Guide tubes 810 incorporate through channels 850 as shown in guide tube 810A in FIG. 8A, recessed 852 as shown in guide tube 810B in FIG. 8B, serrations 856 as shown in guide tube 810C in FIG. 8C, or any other feature or combination of features that form a weak point that allows the guide tube to be split or peeled apart after withdrawal of the guide tube from around the electrode carrier or the lead connecting the electrode carrier to stimulator unit 120 (FIG. 1). These could be single or multiple features allowing the guide tube to be split into one or more separate pieces to facilitate removal of the guide tube after implantation.

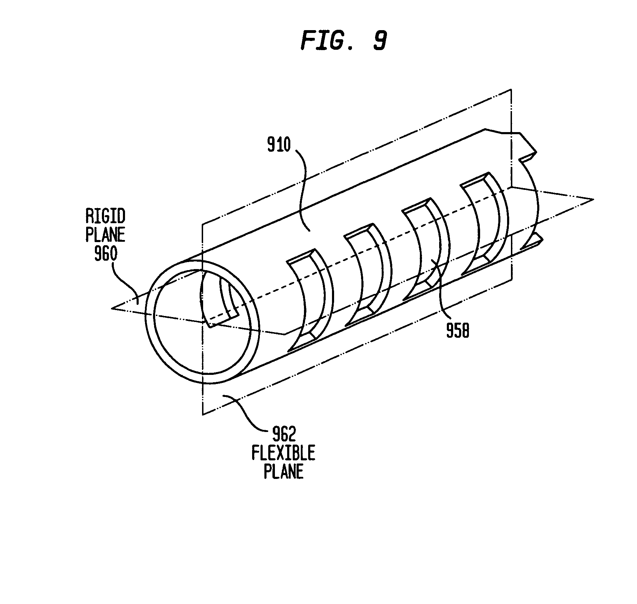

FIG. 9 is a perspective view of electrode assembly guide tube in accordance with one embodiment of the present invention. This embodiment of the guide tube, referred to herein as guide tube 910, is configured to be flexible only in a selected direction. There are two longitudinal planes 960, 962 extending through guide tube 902. On opposing sides of guide tube 902 there are longitudinally-spaced slots each of which is approximately centered on longitudinal plane 960. As a result, guide tube 902 is flexible about plane 902, referred to as flexible plane 902, and is not flexible about longitudinal plane 960, referred to as rigid plane 960.

This embodiment of the guide tube enables the audiologist to better control the electrode assembly. For example, guide tube 910 enables an audiologist to hold the electrode carrier (not shown) straight, and after insertion of the electrode carrier, guide tube 902 is withdrawn, remaining around the carrier or lead wires. At that point, guide tube 902 may be rotated and bent around flexible plane 902. As such, guide tube 902 also serves as a protective sheath around the lead, protecting it from external impact, etc.

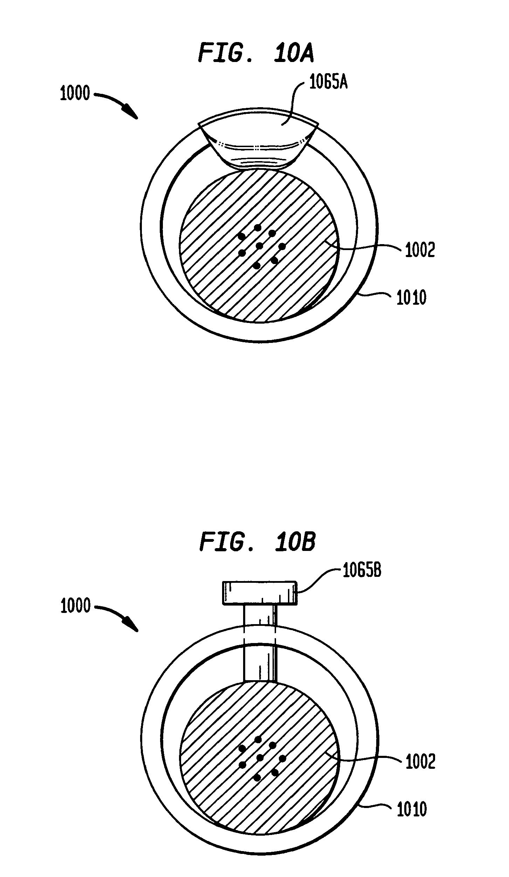

In the embodiment shown in FIGS. 10A and 10B, an embodiment of guide tube 310, referred to herein as guide tube 1010, is configured to incorporate a "brake" 1065 that holds an embodiment of electrode carrier 118, referred to herein as electrode carrier 1002, in place within the guide tube to prevent the carrier from displacing or rotating within the guide tube during transportation and handling. Once guide tube 1010 and electrode carrier 1002 are implanted, brake 1065 may be removed or adjusted so that the carrier may be advanced and the guide tube withdrawn. This ensures electrode carrier 1002 is in the correct position and orientation upon insertion.

In the embodiment illustrated in FIG. 10A, brake 1065A is implemented as a simple silicone flap on proximal end 1020 of guide tube 1010 that mates with and applies pressure to electrode carrier 1002 when the carrier is disposed in lumen 1018 of the guide tube so as to prevent relative displacement. Once guide tube 1010 is properly positioned in cochlea 140, flap 1065A may be lifted thereby allowing electrode carrier 1002 to be slidingly advanced through guide tube 1010.

In the embodiment illustrated in FIG. 10B, a brake 1065B is implemented as a separate peg that clamps electrode carrier 1002 to guide tube 1010. Once the electrode assembly 1000 is in position in cochlea 140, peg 1065B can be removed thereby allowing electrode carrier 1002 to be slidingly advanced through guide tube 1010.

As one of ordinary skill in the art would appreciate, other implementations of brake 665 may be utilized depending on the particular application.

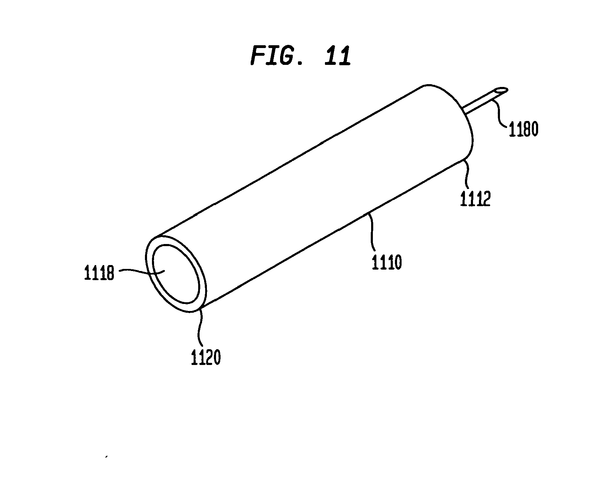

FIG. 11 is a perspective view of an alternative embodiment of a guide tube of the present invention, referred to herein as guide tube 1110. Guide tube 1110 has a distal end 1112, a proximal end 1120 and a lumen 1118 extending longitudinally through guide tube 1110. In this embodiment, a surgical tool 1180 is secured to distal end 1112 of guide tube 1110 to incise the round window membrane or endosteum immediately prior to insertion reducing the risk of leaking perilymph. In such an embodiment, surgical tool 1180 is a rigid, sharp cutting surface similar to that found at the end of a syringe needle. This enables such a tip to perforate the round window membrane during insertion (in the one action). Guide tube 1110 may then continue to be inserted utilizing, for example, image guided surgery, so that the tip does not contact other structures of cochlea 140.

Advantageously, perforation of the round window (or exposed endosteum for a cochleostomy insertion) in this manner eliminates the delay traditionally experienced between perforation and insertion of the electrode carrier. Immediate insertion after perforation prevents perilymph from escaping, and prevents blood, bone dust or other foreign matter from entering the cochlea. Any one of such occurrences may be detrimental to preservation of residual hearing.

The inventor has determined that successful implantation of a perimodiolar carrier such as those described above relies in part on the accurate positioning of the tip of the electrode carrier within cochlea 140 prior to the bending of the electrode carrier. Since the electrode carrier begins to bend upon it exiting the guide tube, the inventor concluded that the accurate position of the distal end of the guide tube from which the electrode carrier exists determines whether the perimodiolar carrier is likely to be successfully and atraumatically implanted. Incorrect positioning may result in trauma to the cochlea due to, for example, the uncontrolled impact of the electrode carrier with modiolus 212 at an acute-to-substantially orthogonal angle, or an unintended fold over of the tip of the electrode carrier.

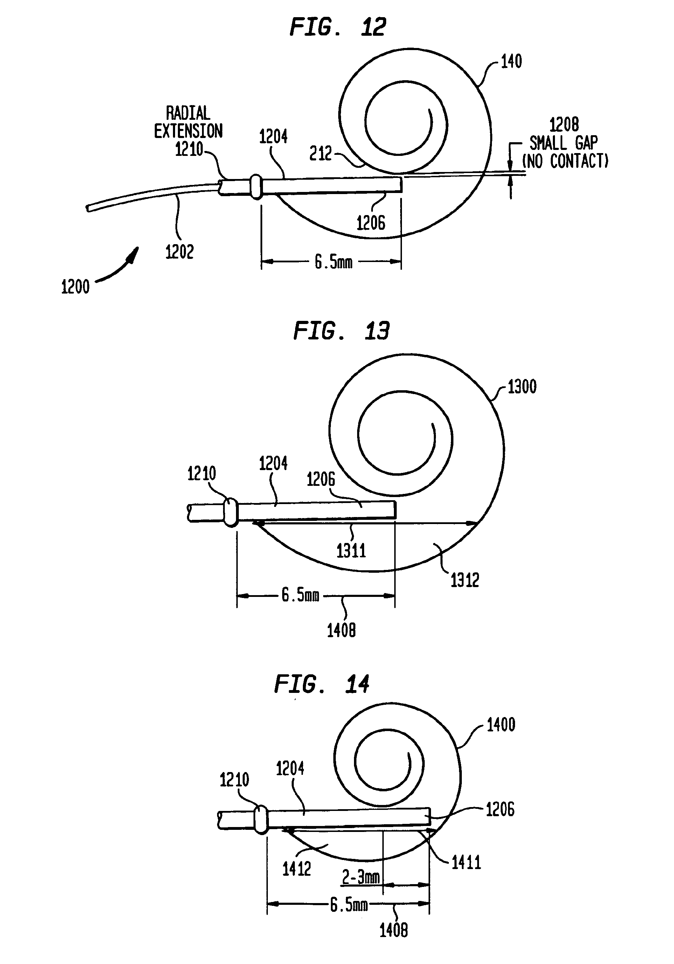

FIG. 12 is a side view of a schematic representation of cochlea 140 with a representative guide tube/electrode carrier arrangement 1200 implanted therein. To ensure minimal contact trauma between a guide tube 1204 and modiolus 212 of cochlea 140, the distal end 1206 of guide tube 1204 preferably does not come into contact with the modiolus; that is there is a small gap 1208 between modiolus 212 and guide tube 1204. In other words, guide tube 1204 is preferably implanted in cochlea 140 so as to be positioned slightly mid-scala. Additionally, an ideal position of guide tube 1204 prior to advancing electrode carrier 1202 is as close as possible and tangential to modiolus 212 to allow a smooth deployment of the electrode carrier with minimal contact trauma. Contact between guide tube 1204 and modiolus 212 is difficult to avoid however.

In addition, the final position of distal end 1206 of guide tube 1204 in relation to modiolus 212 is also dependent on the size of the cochlea. In FIG. 12 cochlea 140 is an average cochlea. The exemplary embodiment of guide tube 1204 is 6.5mm from radial extension 1210 to distal end 1206. In such an average cochlea, when guide tube 1204 is inserted into the cochlea until radial extension 1210 contacts cochlea 140, distal end 1206 is located at the first turn of the cochlea. As such, carrier 1202 may be successfully implanted with minimal trauma to cochlea 140.

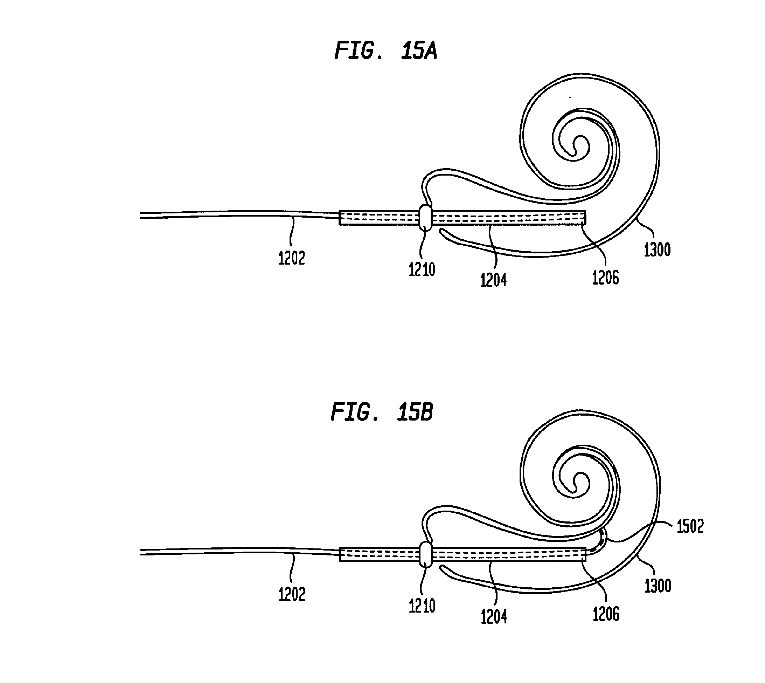

Referring to FIGS. 14 and 15, guide tube 1204 is shown inserted into two cochlea 1300 and 1400. The length 1311 of basal region 1312 of cochlea 1300 is approximately 2-3mm longer than basal length 1411 of cochlea 1400, with average cochlea 140 illustrated in FIG. 12 having a basal length somewhere between these two cochlea. This 2 to 3 mm variation in basal length results in electrode carrier 1202 exiting guide tube 1204 in basal region 1312 prior to the first turn of cochlea 1300, thereby increasing the likelihood that the permodiolar electrode carrier 1202 will cause contact trauma to modiolus 212. This is illustrated in FIGS. 15A and 15B. In FIG. 15A, guide tube 1204 and carrier 1202 are implanted into cochlea 1300 until radial extension 1210 contacts the cochlea, at which point distal end 1206 is located at a position which is before the first turn of the cochlea. As shown in FIG. 15B, when perimodiolar electrode carrier 1202 is deployed from distal end 1206 of guide tube 1204, distal end 1502 of permodiolar carrier 1202 curves to contact modiolus 212 of cochlea 1300 at an acute-to-substantially orthogonal angle thereby causing trauma to the cochlea.

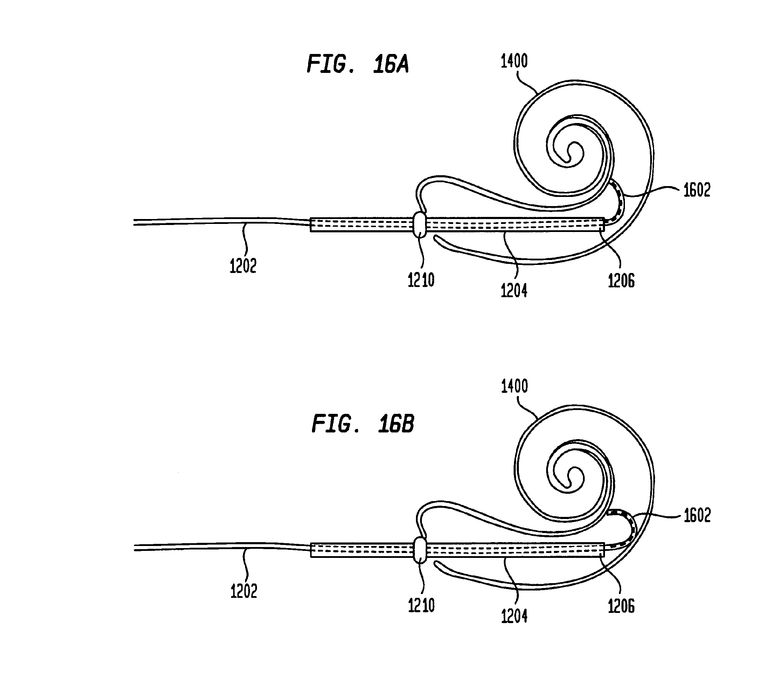

On the other hand, when guide tube 1204 is inserted into relatively small cochlea 1400, distal end 1206 of guide tube 1204 extends beyond the first turn of cochlea 1400. This may cause the distal end of guide tube 1204 to contact lateral sidewall 218 of cochlea 1400, possibly causing trauma. Furthermore, as perimodiolar carrier 1202 extends from distal end 1206 of guide tube 1204, the distal end of carrier 1202 folds-over upon itself. This is illustrated in FIGS. 16A and 16B, in which distal end 1602 of electrode carrier 1202 extending from distal end 1206 of guide tube 1204 curves to contact modiolus 212 such that further extension of electrode carrier 1202 results in distal end 1602 to fold over as shown in FIG. 16B.

Unfortunately, the size of the human cochlea in one recipient is often not the same as the size of the cochlea in another recipient. Such patient variability increases the likelihood that a guide tube configured for one cochlea size may not be successfully used to implant an electrode carrier in many other recipients.

Certain embodiments of the present invention comprise a guide tube that may be used to successfully implant a perimodiolar electrode carrier and other electrode carriers in cochlea of different sizes with minimal potential for tip fold-over and trauma to modiolus 212. Specifically, the following embodiments of the guide tube of the present invention overcome the wide variations in cochlea size by comprising a flexible distal end region and a rigid guide tube body. The distal end region is sufficiently flexible to adjust its shape in response to forces applied to the distal end region by the electrode carrier positioned within its lumen. By adjusting; that is, bending, to an extent dictated by the amount the electrode carrier extends through the distal end region, and whether the distal end region abuts a cochlea structure, particularly, the modiolus. As such, the distal end region of the guide tube serves as a deployment ramp for the pre-curved electrode carrier causing the electrode carrier to follow an optimized trajectory as it comes into contact with the modiolus. Specifically, the electrode carrier follows a trajectory that is tangential with the surface of the modiolus 212 when it comes into contact with the modiolus. This minimizes contact trauma and reduces the likelihood of tip fold over, and ensures that a single electrode/insertion technique may be implemented to accommodate the full range of cochlea sizes.

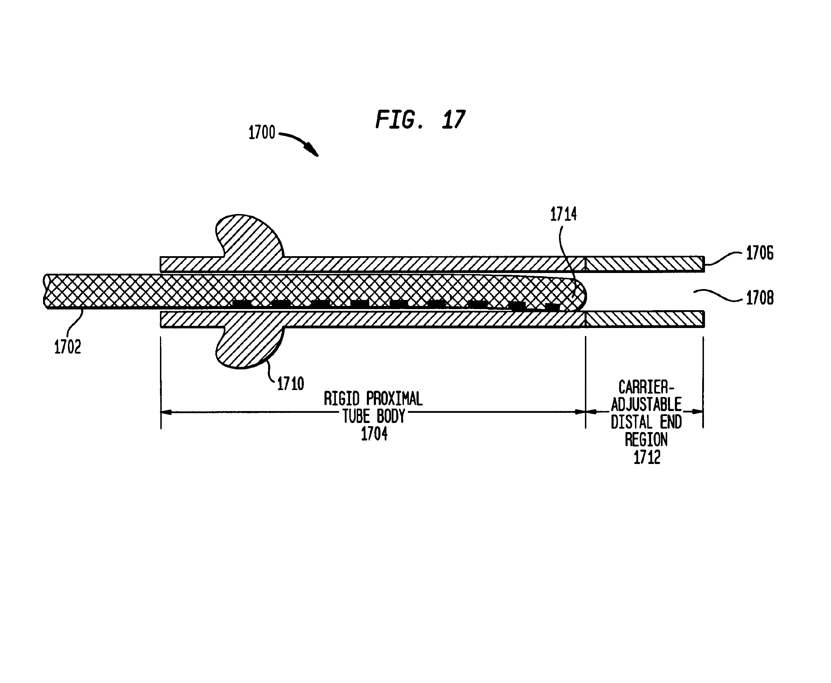

FIG. 17 is a perspective view of an embodiment of a guide tube of the present invention, referred to herein as guide tube 1700. In FIG. 17, guide tube 1700 has a relatively rigid proximal tube body 1704 and a unitary or integrated distal end region 1710. Distal end region 1710 is more malleable, or softer than the body region 1704 to reduce potential damage to cochlea 140. This may be achieved by forming distal region 1710 with a soft material such as silicone, Eurathane, PEBAX, etc.

In another embodiment, a length of guide tube 1700 may be constructed from different layers; that is, a laminate, of polymer materials of differing stiffness. For example, a section of distal end region 1712 may then have specific layer(s) removed using a chemical or laser etching process, leaving only flexible layers for the distal region and a more rigid proximal body region. Alternatively, a polymer or silicone material having a high temperature cure may be used such that localized heating may be applied during the curing process such that the tip is not fully cured and hence softer/more flexible.

The dimensions of an attached soft distal end region 1712 would typically be of equivalent inner diameter and outer diameter to guide tube body region 1704. The length would be such that its rigidity is sufficient to maintain an associated electrode carrier 1702 in a straight configuration, yet long enough to safely deflect/flex when inserted into cochlea 140. In one embodiment, this length is between 2 and 4mm.

Generally, the embodiments of guide tube 1700 described below each comprise a relatively rigid or stiff proximal tube body 1704 that does not significantly flex or bend in response to forces applied by a perimodular electrode carrier positioned 1702 in the guide tube lumen 1708. The guide tube 1700 further comprises an integrated or unitary distal end region 1712 that is sufficiently flexible to be responsive to the bending forces applied by electrode carrier 1702. Carrier-responsive distal end region 17012 is sufficiently stiff or rigid to prevent the guide tube 1700 from folding upon itself in response to the bending forces applied by the carrier. In other words, carrier-responsive distal end region 1712 bends in the area in which the carrier forces are applied while otherwise remaining relatively straight. Thus, as carrier 1702 is initially extended into distal end region 1712, distal end region 1712 will bend slightly in the vicinity of the bending force applied to the proximal end of the distal end region. In other words, the portion of distal end region 1712 which is subject to the bending forces applied by carrier 1702 is the portion of distal end region 1704 which bends. The remaining portion of distal end region 1704 generally does not bend as it has some inherent stiffness to maintain its form and is otherwise not subjected to bending forces from a source other than the carrier.

As electrode carrier 1702 is further longitudinally extended into guide tube 1700, the carrier further enters distal end region 1712. In response, distal end region 1712 bends further as the force applied by the carrier 1702 is applied further along the cantilevered distal end region. In other words, the portion of distal end region 1712 that bends in response to the carrier increases. As electrode carrier 1702 continues to extend into distal end region 1704, the portion of distal end region that bends expands and advances accordingly until the distal end region comes into contact with modiolus 212. As electrode carrier 1702 continues to travel through and exit distal end region 1712 the electrode carrier bends around the modiolus 212. Thus, distal end region 1712 serves to prevent electrode carrier 712 from abutting modiolus 212 prematurely and at an angle that would cause trauma to the modiolus. Distal end region 1712 further serves to prevent fold-over of electrode carrier 1702 by restricting its ability to bend to such a degree that it curls. In doing so, distal end region 1712 serves as an exit ramp that places electrode carrier 1702 on a trajectory that is sufficiently tangential with the modiolus that the electrode carrier atraumatically contacts modiolus 212 and, as carrier further extends beyond the distal end region, the carrier continues to bend and wrap itself around the modiolus. Thus, distal end region 1712 provides an optimized departure trajectory path for the carrier as the carrier exits the distal opening of the guide tube. This enables the electrode carrier to gently make contact with the modiolus at an angle that promotes safe insertion of the electrode carrier, while concurrently maintaining a tight radius of curvature that enables the electrode carrier to hug the modiolus once fully inserted.

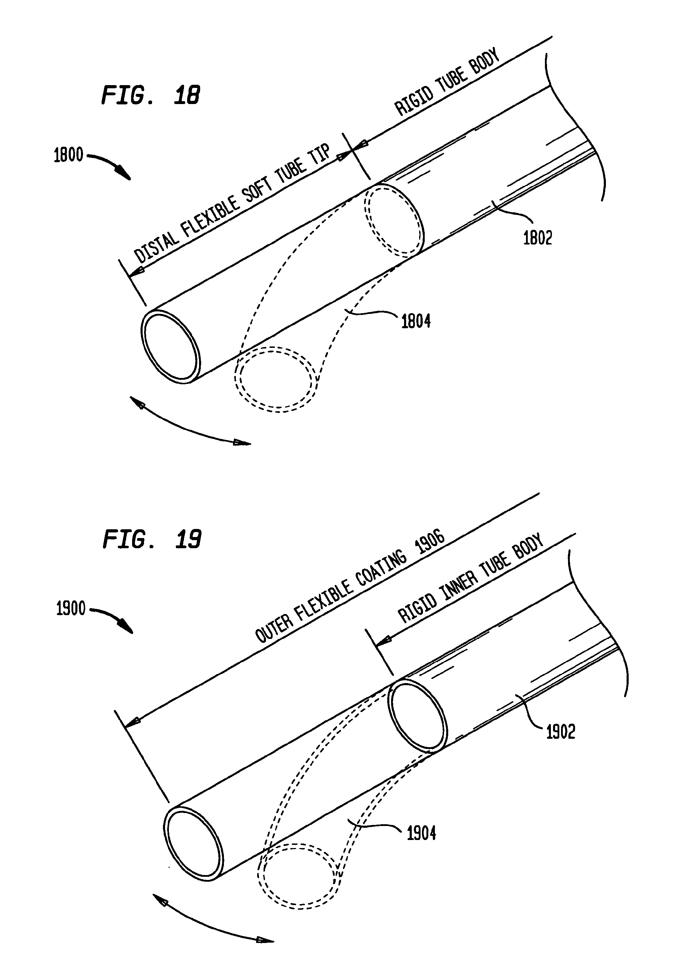

FIGS. 18-24 are perspective views of alternative embodiments of guide tube 1700 of the present invention. FIG. 18 is a perspective view of one embodiment of the guide tube of the present invention, referred to herein as guide tube 1800. Guide tube 1800 comprises a rigid tube body 1802 and a carrier-adjustable distal end region 1804 that is soft and flexible relative to rigid tube body 1802 such that distal end region 1804 bends in response to forces applied by electrode carrier that do cause rigid tube body 1802 to bend. In one embodiment, guide tube 1800 is formed of silicone. In one embodiment, this flexible distal end region 1804 is a separately manufactured element that is attached to rigid tube body 1802. Such an attachment may be by abutting the body 1802 and distal end region 1804. Alternatively, the attachment may be attained with an overlap joint.

FIG. 19 is a perspective view of one embodiment of the guide tube of the present invention, referred to herein as guide tube 1900. Guide tube 1900 comprises a rigid distal body 1802 formed of a rigid inner tube. In this embodiment, an outer flexible coating 1902 is applied to rigid inner tube body 1902. Carrier-bendable distal end region 1904 comprises coating 1906 that distally extends beyond rigid inner tube 1902.

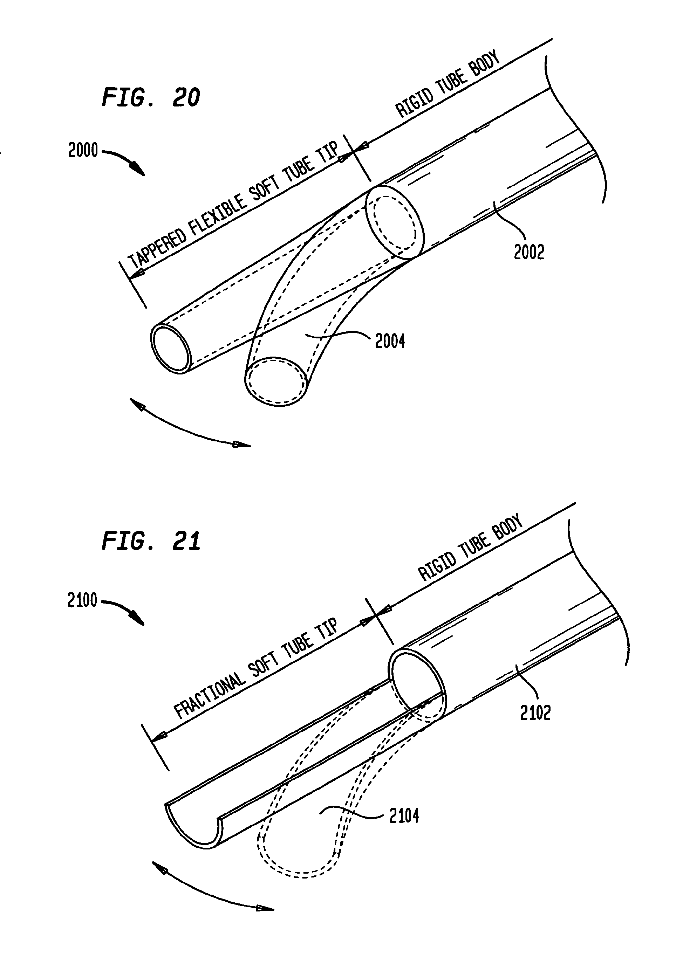

FIG. 20 is a perspective view of one embodiment of the guide tube of the present invention, referred to herein as guide tube 2000. Guide tube 2000 comprises a rigid distal body 2002 and a tapered flexible soft tube tip forming distal end region 2004. In this embodiment, carrier-bendable distal end region 2004 comprises a coating that is applied to rigid inner tube 2002 as described above with reference to FIG. 19 or, alternatively, a flexible silicone tip as described above with reference to FIG. 18. This provides additional flexibility, which can be controlled via the taper.

FIG. 21 is a perspective view of one embodiment of the guide tube of the present invention, referred to herein as guide tube 2100. Guide tube 2100 comprises a rigid tube body 2102 and a tapered fractional soft tube tip forming distal end region 2104. In this embodiment, carrier-bendable distal end region 2104 may comprise a coating that is applied to rigid inner tube 2102 as described above with reference to FIG. 19 or, alternatively, a flexible silicone tip as described above with reference to FIG. 18. As shown in FIG. 21, the fractional soft tube tip extends around the electrode carrier (not shown) to be adjacent to at least the modiolar side of electrode carrier. This embodiment provides a further advantage of providing a smaller profile of distal end region 2104 to facilitate the atraumatic insertion of the guide tube through, for example, the round window.

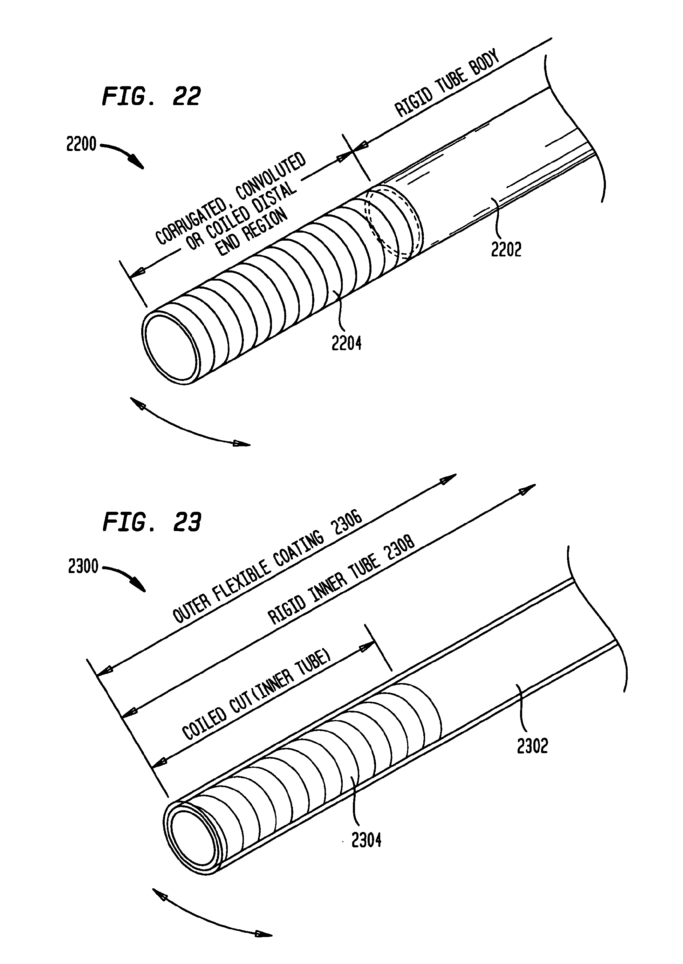

FIG. 22 is a perspective view of one embodiment of the guide tube of the present invention, referred to herein as guide tube 2200. Guide tube 2200 comprises a rigid distal body 2202 and a corrugated, convoluted or coiled tube forming distal end region 2204. In one embodiment, carrier-bendable distal end region 2204 is formed from a flexible silicone tip as described above with reference to FIG. 18 with a coiled cut in the rigid inner tube such that the outer flexible coating (Fig. 19) provides some integrity whilst still being flexible.

FIG. 23 is a perspective view of one embodiment of the guide tube of the present invention, referred to herein as guide tube 2300. Guide tube 2300 comprises a rigid inner tube 2308 forming rigid distal body 2302, and a distal end region 2304 formed of rigid inner tube 2308 with a coiled cut in the rigid inner tube. An outer flexible coating 2306 is applied to rigid inner tube 2300 to provide structural integrity to distal end region 2304.

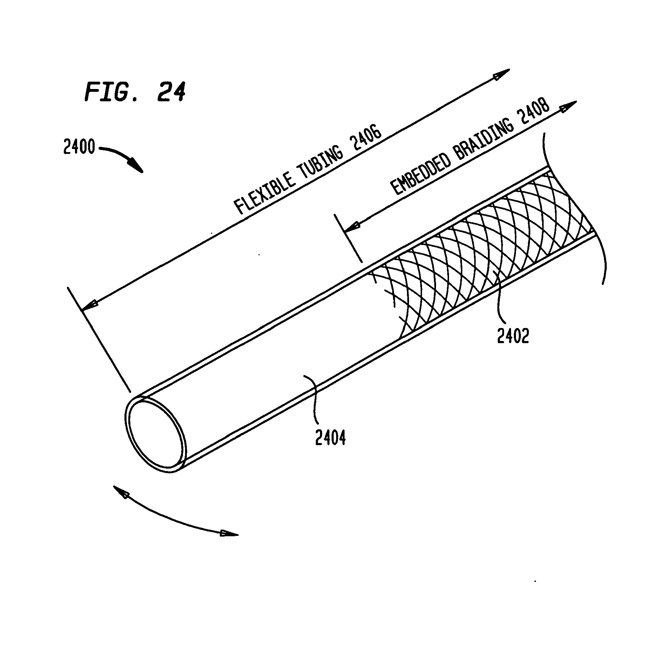

FIG. 24 is a perspective view of one embodiment of the guide tube of the present invention, referred to herein as guide tube 2400. Guide tube 2400 comprises a flexible tubing 2406 forming distal end region 2404 and rigid guide tube body 2402. Rigid guide tube body 2402 also comprises a braid 2408 embedded into flexible tubing 2406 such that rigid guide tube 2400 is sufficiently rigid to withstand the environment.

As one of ordinary skill in the art would appreciate, the above embodiments are just a few examples of how one may implement the guide tube of the present invention. Furthermore, any of the above or other embodiments of the guide tube of the present invention may be formed of one or more of silicones, polyurethanes, polyimides, etc.

In use, any of the above or other embodiments of the guide tube of present invention provide significant advantages over convention perimodiolar electrode carriers. Typically, conventional perimodiolar electrode carriers are based on a stylet design for inserting the electrode carrier a specified depth past the round window or cochleostomy before advancing the electrode off the stylet. Such conventional approaches fail to compensate for variations in cochlea size which, as noted, varies considerably in a population. In contrast, embodiments of the present invention specifically address variations in cochlea size and compensate for these variations without the requirement for high resolution pre-operative imaging or other techniques to ensure that a particular electrode carrier is matched to a particular cochlea. This provides significant benefit, efficiency and cost savings, and more importantly reduces the potential risk of damaging residual hearing by inserting an electrode which may not be optimized for a particular cochlea size.

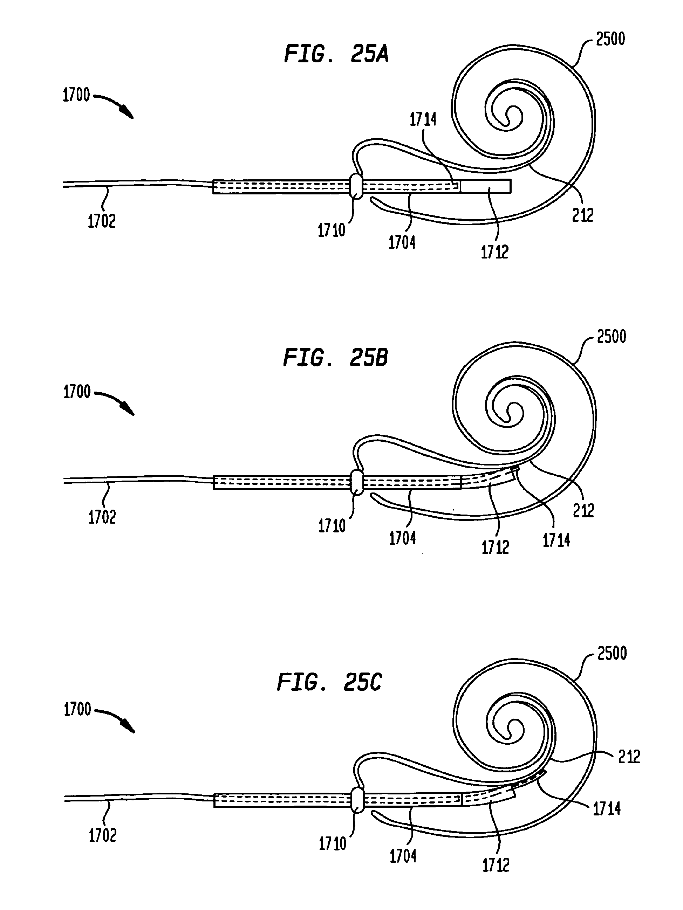

FIGS. 25A-25C are perspective views of one embodiment of guide tube 1700 inserted into an average size cochlea, in accordance with one embodiment of the present invention. In FIG. 25A, the electrode carrier 1702 is located in rigid tube body 1704 and not in distal end region 1712. In FIG. 25B, electrode carrier 1702 is extended through guide tube 1700 such that distal end 1714 of electrode carrier 1702 is positioned exiting the distal end of the carrier-adjustable distal end region 1712 of guide tube 1700. As shown in FIG. 25B, the force applied by carrier 1702 on guide tube 1700 causes carrier-adjustable distal end region 1712 to bend slightly to come into contact with modiolus 212 of cochlea 2500. As such, distal end region 1712 of guide tube 1700 becomes a deployment ramp causing the electrode carrier to follow an optimized trajectory as it exits the distal end of the guide tube 1700 so that the electrode carrier follows a trajectory that is substantially tangential with the surface of modiolus 212 when it comes into contact with the modiolus, or at least sufficiently tangential (that is, at an acute angle) with the modiolus surface that the contact is atraumatic. This minimizes contact trauma and reduces the likelihood of tip fold over. This is shown in FIG. 25C in which distal end 1714 of carrier 1702 travels along the surface of modiolus 212 without causing trauma due to the trajectory that the carrier exited guide tube 1700.

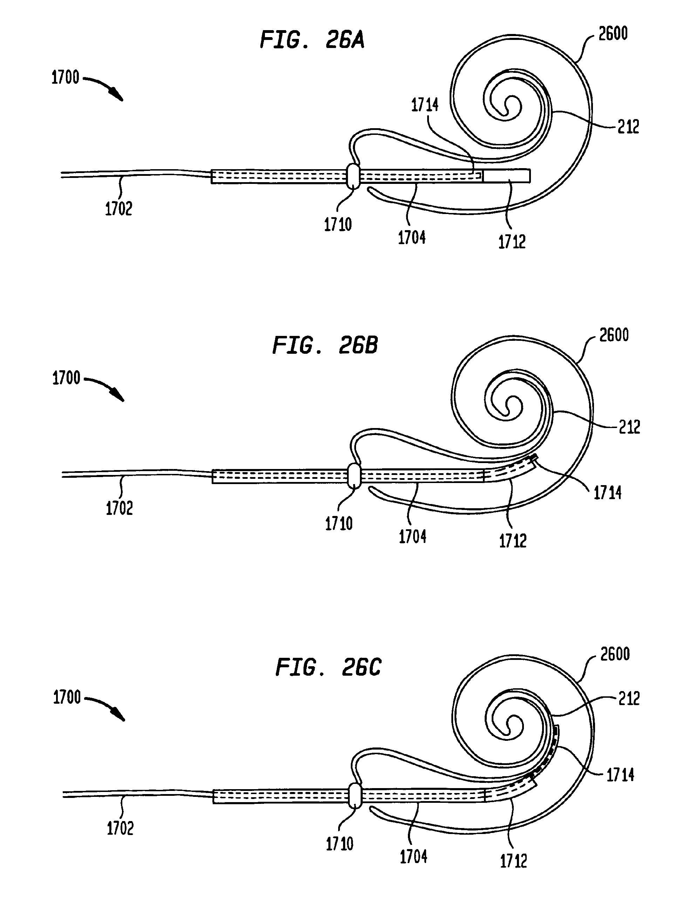

FIGS. 26A-26C illustrate the same operation and results of the guide tube 1700 when inserted into a relatively smaller cochlea 2600. As shown in FIG. 26B, because distal end region 1712 of guide tube 1700 extends beyond the first turn of cochlea 2600, when electrode carrier 1702 causes the distal end region to bend, the distal end region bends slightly further that it may have bent in cochlea 2500. The result, however, is the same; that is distal end region 1712 of guide tube 1700 becomes a deployment ramp causing the electrode carrier to follow an optimized trajectory as it exits the distal end of the guide tube 1700 so that the electrode carrier follows a trajectory that is substantially tangential with the surface of modiolus 212 so that its contact with the modiolus is sufficiently tangential (that is, at an acute angle) with the modiolus surface that the contact is atraumatic.

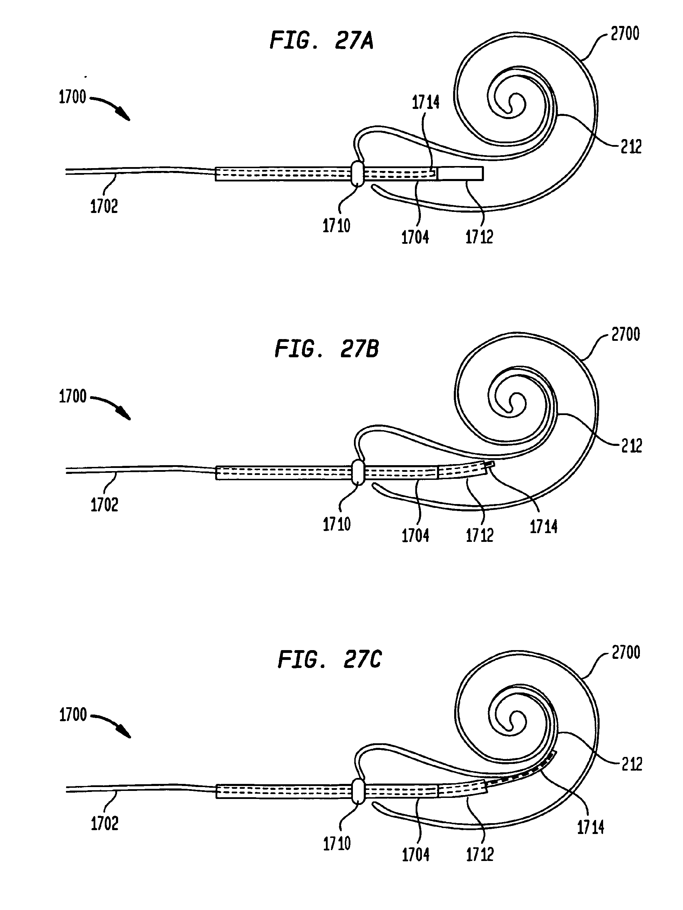

FIGS. 27A-27C illustrate the same operation and results of the guide tube 1700 when inserted into a relatively larger cochlea 2700. As shown in FIG. 26B7 because distal end region 1712 of guide tube 1700 is located in the basal region of the cochlea before the first turn, when electrode carrier 1702 causes the distal end region to bend, the distal end region bends minimally because the guide tube is substantially parallel with the adjacent cochlea structure. The result, however, is the same; that is distal end region 1712 of guide tube 1700 becomes a deployment ramp causing the electrode carrier to follow an optimized trajectory as it exits the distal end of the guide tube 1700 so that the electrode carrier follows a trajectory that is substantially tangential with the surface of modiolus 212 so that its contact with the modiolus is sufficiently tangential (that is, at an acute angle) with the modiolus surface that the contact is atraumatic.

Thus, the carrier-adjustable distal end region of the guide tube of the present invention initially reduces potential damage to the cochlea when the guide tube is initially inserted through the round window or cochleostomy. Once inserted to its predetermined depth in the basal section of the cochlea the carrier is advanced and the distal end region then acts as a ramp, guiding the tip of the electrode carrier and regardless of the size of the cochlea.

More specifically, the distal end region is configured to curve under the force of the advancing pre-curved electrode carrier, which is typically held straight in the rigid tube body, such that the inherent flexibility of the distal end region becomes a ramp that gradually orients itself with the curvature of the modiolus, thus preventing electrode carrier impact trauma and the potential for tip foldover. It also accommodates the infinite variation in cochlea size from the largest to the smallest, using a single insertion depth.

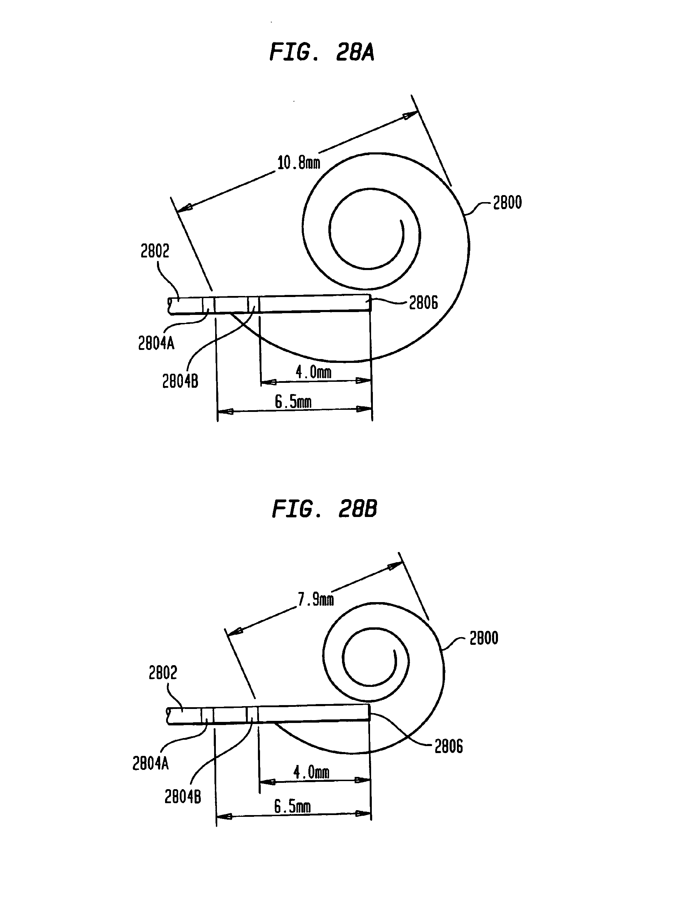

FIGS. 28A and 28B are side views of a relatively larger cochlea 2800 with an alternative embodiment of a guide tube having position markers 2804A and 2804B to facilitate insertion of the guide tube.

Although the present invention has been fully described in conjunction with several embodiments thereof with reference to the accompanying drawings, it is to be understood that various changes and modifications may be apparent to those skilled in the art. Such changes and modifications are to be understood as included within the scope of the present invention as defined by the appended claims, unless they depart therefrom.

## Claims

1. An electrode assembly (300) for implantation in a cochlea (140) of a recipient, comprising:
a flexible, elongate electrode carrier (302) configured to have a spiral configuration such that bias forces are exerted by said electrode carrier (302) when arranged in a non-spiral position; and
an elongate guide tube (310, 810) comprising a rigid tube body, having interior dimensions defining a lumen for slidingly retaining the carrier (302), and exterior dimensions configured to allow said guide tube (310, 810) to be inserted through a cochleostomy region (122) in the recipient's cochlea (140),
**characterized in that**
the guide tube (310, 810) has one or more regions of weakness (850, 852, 856) configured to enable at least a portion of the guide tube (310, 810) to be removed.

2. The electrode assembly of claim 1, wherein a distal end region is configured as a fraction of a tube.

3. The electrode assembly of claim 1, wherein the surface of a distal end region is configured as a corrugated or convoluted surface.

4. The electrode assembly of claim 1, wherein the rigid guide tube comprises a braid structure configured to provide rigidity to the rigid guide tube.

5. The electrode assembly of claim 1, wherein the carrier is pre-curved to attain a perimodiolar position in the scala tympani of the cochlea when implanted.

6. The electrode assembly of claim 1, wherein the carrier is configured to be implanted into the scala tympani, and to minimally interfere with the natural hydrodynamic nature of the cochlea.

7. The electrode assembly of claim 1, wherein the guide tube is constructed from one or more bioresorbable materials.

8. The electrode assembly of claim 1, wherein the guide tube further comprises a radially-extending extension configured to indicate when the guide tube has been inserted to a predetermined depth into the cochlea.

9. The electrode assembly of claim 1, wherein a distal end of the guide tube and an end of the carrier are configured to interact to maintain a relative longitudinal position of the carrier and guide tube.

10. The electrode assembly of claim 1, wherein the one or more regions of weakness are formed by disposing one or more serrations longitudinally along the guide tube.

11. The electrode assembly of claim 1, wherein the guide tube further comprises a releasable brake constructed and arranged to hold the carrier in place within the guide tube to prevent the carrier from displacing or rotating within the guide tube.

## Patentansprüche

1. Elektrodenanordnung (300) zur Implantation in eine Cochlea (140) eines Empfängers, die Folgendes umfasst:
einen flexiblen, langgestreckten Elektrodenträger (302), der konfiguriert ist, eine Spiralkonfiguration aufzuweisen, so dass Vorspannungskräfte durch den Elektrodenträger (302) ausgeübt werden, wenn er sich nicht in einer Spiralposition befindet; und
eine langgestreckte Führungsröhre (310, 810), die einen starren Röhrenkörper umfasst, mit Innenabmessungen, die ein Lumen definieren, um den Träger (302) gleitend zu halten, und mit äußeren Abmessungen, die konfiguriert sind, es der Führungsröhre (310, 810) zu erlauben, durch einen Cochleostomiebereich (122) in die Cochlea (140) eines Empfängers eingeführt zu werden,
**dadurch gekennzeichnet, dass**
die Führungsröhre (310, 810) ein oder mehrere Schwachstellenbereiche (850, 852, 856) aufweist, die konfiguriert sind, zuzulassen, dass zumindest ein Teil der Führungsröhre (310, 810) entfernt wird.

2. Elektrodenanordnung nach Anspruch 1, wobei ein distaler Endbereich als ein Bruchteil einer Röhre konfiguriert ist.

3. Elektrodenanordnung nach Anspruch 1, wobei die Oberfläche des distalen Endbereichs als eine gewellte oder gefaltete Oberfläche konfiguriert ist.

4. Elektrodenanordnung nach Anspruch 1, wobei die starre Führungsröhre eine Flechtstruktur umfasst, die konfiguriert ist, die starre Führungsröhre zu versteifen.

5. Elektrodenanordnung nach Anspruch 1, wobei der Träger vorgekrümmt ist, um eine perimodiolare Position in der Skala Tympani der Cochlea zu erreichen, wenn er implantiert ist.

6. Elektrodenanordnung nach Anspruch 1, worin der Träger konfiguriert ist, in die Skala Tympani implantiert zu werden, und um die natürliche hydrodynamische Beschaffenheit der Cochlea nur minimal zu beeinflussen.

7. Elektrodenanordnung nach Anspruch 1, wobei die Führungsröhre aus einem oder mehreren bioresorbierbaren Materialien konstruiert ist.

8. Elektrodenanordnung nach Anspruch 1, wobei die Führungsröhre weiterhin eine sich radial erstreckende Erweiterung umfasst, die konfiguriert ist, anzuzeigen, wann die Führungsröhre bis zu einer vorbestimmten Tiefe in der Cochlea eingeführt wurde.

9. Elektrodenanordnung nach Anspruch 1, wobei ein distales Ende der Führungsröhre und ein Ende des Trägers konfiguriert sind, so zusammenzuwirken, dass sie eine relative longitudinale Position des Trägers und der Führungsröhre beibehalten.

10. Elektrodenanordnung nach Anspruch 1, wobei die eine oder mehr Schwachstellenbereiche ausgebildet werden, indem eine oder mehrere Einschnitte longitudinal entlang der Führungsröhre angeordnet werden.

11. Elektrodenanordnung nach Anspruch 1, wobei die Führungsröhre weiterhin eine lösbare Bremse umfasst, die konstruiert und angeordnet ist, um den Träger in der Führungsröhre an Ort und Stelle zu halten, um zu verhindern, dass der Träger in der Führungsröhre sich verschiebt oder rotiert.

## Revendications

1. Ensemble d'électrodes (300) destinées à être implantées dans une cochlée (140) d'un patient, comprenant :
un support d'électrode souple allongé (302), configuré de façon à avoir une configuration en spirale telle que des forces de poussée sont exercées par ledit support d'électrode (302) lorsqu'il est disposé dans une position qui n'est pas en spirale ; et
un tube de guidage allongé (310, 810) comprenant un corps de tube rigide, ayant des dimensions intérieures définissant une lumière pour maintenir le support (302) de façon coulissante et des dimensions extérieures configurées pour permettre l'insertion dudit tube de guidage (310, 810) à travers une région de cochléostomie (122) dans la cochlée du patient (140),
**caractérisé en ce que**
le tube de guidage (310, 810) comporte une ou plusieurs régions de faiblesse (850, 852, 856) configurées de façon à permettre le retrait d'au moins une partie du tube de guidage (310, 810).

2. Ensemble d'électrodes selon la revendication 1, dans lequel une région d'extrémité distale est configurée sous la forme d'une fraction de tube.

3. Ensemble d'électrodes selon la revendication 1, dans lequel la surface d'une région d'extrémité distale est configurée sous la forme d'une surface ondulée ou à circonvolutions.

4. Ensemble d'électrodes selon la revendication 1, dans lequel le tube de guidage rigide comprend une structure en tresse configurée pour conférer la rigidité au tube de guidage rigide.

5. Ensemble d'électrodes selon la revendication 1, dans lequel le support est incurvé à l'avance afin d'atteindre une position périmodiolaire dans la rampe tympanique de la cochlée, une fois implanté.

6. Ensemble d'électrodes selon la revendication 1, dans lequel le support est configuré de façon à être implanté dans la rampe tympanique et à gêner au minimum la nature hydrodynamique naturelle de la cochlée.

7. Ensemble d'électrodes selon la revendication 1, dans lequel le tube de guidage est construit à partir d'un ou plusieurs matériaux biorésorbables.

8. Ensemble d'électrodes selon la revendication 1, dans lequel le tube de guidage comprend en outre une région s'étendant radialement, configurée pour indiquer que le tube de guidage a été inséré dans la cochlée à une profondeur prédéterminée.

9. Ensemble d'électrodes selon la revendication 1, dans lequel une extrémité distale du tube de guidage et une extrémité du support sont configurées de façon à interagir pour maintenir une position longitudinale relative du support et du tube de guidage.

10. Ensemble d'électrodes selon la revendication 1, dans lequel la ou les régions de faiblesse est(sont) formée(s) en disposant une ou plusieurs dentelures longitudinalement le long du tube de guidage.

11. Ensemble d'électrodes selon la revendication 1, dans lequel le tube de guidage comprend en outre un frein amovible construit et agencé de façon à maintenir le support en place à l'intérieur du tube de guidage pour empêcher le support de se déplacer ou de tourner à l'intérieur du tube de guidage.
